(19) Europäisches Patentamt
European Patent Office
Office européen des brevets

(11) Veröffentlichungsnummer: **0 225 552**
**A2**

(12) # EUROPÄISCHE PATENTANMELDUNG

(21) Anmeldenummer: **86116504.1**

(22) Anmeldetag: **27.11.86**

(51) Int. Cl.⁴: **C 07 D 498/06**, C 07 D 471/06, A 61 K 31/535, A 61 K 31/495, A 61 K 31/55 // (C07D498/06, 265:00, 221:00)

(30) Priorität: **10.12.85 DE 3543513**

(43) Veröffentlichungstag der Anmeldung: **16.06.87 Patentblatt 87/25**

(84) Benannte Vertragsstaaten: **AT BE CH DE ES FR GB IT LI NL SE**

(71) Anmelder: **BAYER AG, Konzernverwaltung RP Patentabteilung, D-5090 Leverkusen 1 Bayerwerk (DE)**

(72) Erfinder: **Schriewer, Michael, Dr., Am Thelen Siefen 1a, D-5068 Odenthal (DE)**
Erfinder: **Grohe, Klaus, Dr., Am Wasserturm 10, D-5068 Odenthal (DE)**
Erfinder: **Zeiler, Hans-Joachim, Dr., Elsbeeker Strasse 46, D-5620 Velbert 15 (DE)**
Erfinder: **Metzger, Karl Georg, Dr., Pahlkestrasse 75, D-5600 Wuppertal 1 (DE)**

(54) **Enantiomerenreine 1,8-verbrückte 4-chinolon-3-carbonsäuren, Verfahren zu ihrer Herstellung sowie diese enthaltende Arzneimittel und ihre Verwendung zur Herstellung von Arzneimitteln.**

(57) Die Erfindung betrifft enantiomerenreine 1,8-verbrückte 4-Chinolon-3-carbonsäuren der Formel (I)

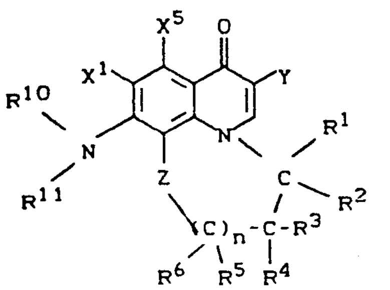

(I)

in der die Symbole $R^1$ bis $R^{10}$, Y, $X^1$, $X^5$, Z und n die in der Beschreibung angegebene Bedeutung haben, ein Verfahren zu ihrer Herstellung sowie ihre Verwendung als Arzneimittel, insbesondere als antibakterielle Mittel in der Human- und Veterinärmedizin.

EP 0 225 552 A2

0225552

BAYER AKTIENGESELLSCHAFT 5090 Leverkusen, Bayerwerk
Konzernverwaltung RP
Patentabteilung Ad/ABc

Enantiomerenreine 1,8-verbrückte 4-Chinolon-3-carbonsäuren, Verfahren zu ihrer Herstellung sowie diese enthaltende Arzneimittel und ihre Verwendung zur Herstellung von Arzneimitteln

Die Erfindung betrifft enantiomerenreine 1,8-verbrückte 4-Chinolon-3-carbonsäuren, ein Verfahren zu ihrer Herstellung sowie ihrer Verwendung als Arzneimittel, insbesondere als antibakterielle Mittel in der Human- und Veterinärmedizin.

Gegenstand der Erfindung sind enantiomerenreine 1,8-verbrückte 4-Chinolon-3-carbonsäuren und Derivate der Formel (I)

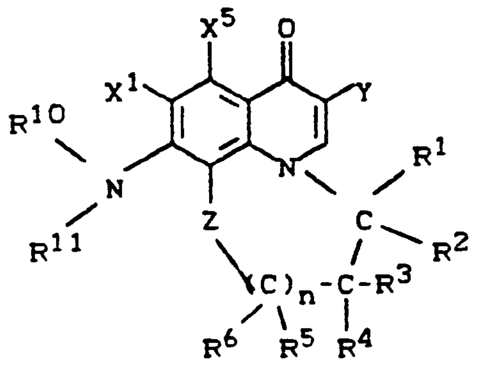

(I)

in der

Y eine Carboxylgruppe, eine Nitrilgruppe, eine Estergruppe $-COOR^7$ oder eine Säureamidgruppe $-CONR^8R^9$ darstellt, wobei $R^7$ für $C_1$-$C_4$-Alkyl steht, $R^8$ und $R^9$ für Wasserstoff oder $C_1$-$C_4$-Alkyl stehen, $R^9$ außerdem gegebenenfalls substituiertes Phenyl sein kann.

$X^1$ für Wasserstoff, Nitro, Alkyl mit 1 bis 3 Kohlenstoffatomen, Halogen, bevorzugt Fluor steht,

$X^5$ Wasserstoff, Halogen oder Methyl sein kann,

$R^{10}$ und $R^{11}$ gemeinsam mit dem Stickstoffatom, an das sie gebunden sind, einen 5- oder 6-gliedrigen heterocyclischen Ring bilden, der als Ringglied zusätzlich die Atome oder Gruppen -O-, -S-, -SO-, $-SO_2-$, $>N-R^{12}$ oder $-CO-\overset{|}{N}-R^{12}$ enthalten kann und der gegebenenfalls an den Kohlenstoffatomen ein- bis dreifach durch $C_1$-$C_4$-Alkyl, gegebenenfalls durch Chlor, Fluor, Brom, Methyl, Phenyl, Hydroxy, Methoxy, Benzyloxy, Nitro oder Piperidino ein- bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und Ethylaminomethyl substituiert sein kann, wobei

$R^{12}$ für Wasserstoff, eine verzweigte oder unverzweigte Alkyl-, Alkenyl- oder Alkinylgruppe mit 1 bis 6 Kohlenstoffatomen, die gegebenenfalls durch ein oder

0225552

zwei Hydroxy-, Alkoxy-, Alkylamino- oder Dialkylminogruppen mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor oder Fluor ein- oder zweifach substituierten Phenacylrest, einen Oxoalkylrest mit bis zu 6 Kohlenstoffatomen steht, ferner einen Rest $COR^{13}$ oder $SO_2R^{14}$ bedeutet, wobei

$R^{13}$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Alkoxy mit 1 bis 4 Kohlenstoffatomen, Amino, Alkylamino oder Dialkylamino mit 1 bis 5 Kohlenstoffatomen im Alkylteil und

$R^{14}$ geradkettiges oder verzweigtes Alkyl mit 1 bis 3 Kohlenstoffatomen darstellt, und

Z für Sauerstoff, einen Aminrest $NR^{15}$ steht, wobei $R^{15}$ Wasserstoff, einen gegebenenfalls durch Halogen, Trifluormethyl, Nitro, Cyano, Hydroxy, Alkoxy bzw.

Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen, einen gegebenenfalls durch Halogen, eine Nitrogruppe, oder Alkylgruppe mit 1-3 Kohlenstoffatomen, eine Alkoxy- bzw. Alkylmercaptogruppe mit jeweils 1-3 Kohlenstoffatomen substituierten Phenylrest bedeutet, sowie ferner einen Acylrest $R^{16}$-CO- bzw. $R^{17}SO_2$- darstellt, wobei $R^{16}$ bzw. $R^{17}$ Alkylreste mit 1-6 Kohlenstoffatomen oder gegebenenfalls substituierte Phenylreste darstellen, sowie ein

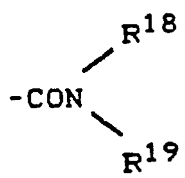

oder

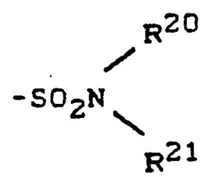

-Rest sein kann, wobei die Reste $R^{18}$ bis $R^{21}$ Wasserstoff, Alkyl mit 1-6 Kohlenstoffatomen oder einen gegebenenfalls substituierten Phenylrest darstellen

und

$R^1$, $R^2$, $R^3$, $R^4$, $R^5$ und $R^6$ für Wasserstoff, eine gegebenenfalls ein- oder mehrfach durch Halogen, insbesondere Chlor oder Fluor, ferner Nitro, Cyano, Hydroxy, Alkoxy, bzw. Alkylmercapto mit 1-3 Kohlenstoffatomen im Alkoholteil substituierte Alkylgruppe mit 1-6 Kohlenstoffatomen, einen gegebenenfalls durch Halogen, Nitro, Alkyl bzw. Alkoxy bzw. Alkylmercapto mit jeweils bis zu 3 Kohlenstoffatomen, Hydroxy, Aryloxy, Arylthio, Cyano oder einen Esterrest mit 1-3

Kohlenstoffatomen im Alkoholteil substituierten Phenylrest, Naphthylrest oder heterocyclischen Rest wie z.B. Thiophen-, Furan-, Pyrrol-, Thiazol-, Pyridin- oder Pyrimidin-Rest stehen mit der Maßgabe, daß $R^1$ und $R^2$ und/oder $R^3$ und $R^4$ und/oder $R^5$ und $R^6$ verschieden sind, sowie $R^2$ mit $R^3$ und/oder $R^4$ mit $R^5$ jeweils mit dem Kohlenstoffatomen, an das sie gebunden sind, einen 3-7 gliedrigen Ring bilden, der gegebenenfalls durch gegebenenfalls substituierte Alkylreste mit 1-3 Kohlenstoffatomen oder gegebenenfalls substituierte Arylreste substituiert ist und

n = 0 oder 1 bedeutet,

und deren pharmazeutisch verwendbare Hydrate, Alkali-, Erdalkali-, Silber- und Guanidiniumsalze, sowie ihre Ester, die eine hohe antibakterielle Wirksamkeit aufweisen.

Sie eignen sich daher als Wirkstoffe für die human- und Veterinärmedizin und vor allem als Zwischenprodukte für derartige Bakterizide.

Bevorzugt sind diejenigen Verbindungen der Formel (1), in denen

Y eine Carboxylgruppe, eine Nitrilgruppe oder eine Estergruppe -$COOR^7$ darstellt, wobei $R^7$ Methyl oder Ethyl sein kann,

$X^1$ für Fluor steht,

$X^5$ für Wasserstoff steht,

$R^{10}$ und $R^{11}$ gemeinsam mit dem Stickstoffatom, an das sie gebunden sind, einen 5- oder 6-gliedrigen heterocyclischen Ring bilden können, der als Ringglied zusätzlich ein Sauerstoffatom oder die Gruppen $>N-R^{12}$ oder $-CO-\overset{|}{N}-R^{12}$ enthalten kann und der gegebenenfalls an den Kohlenstoffatomen ein- bis zweifach durch $C_1$-$C_2$-Alkyl, Cyclohexyl, gegebenenfalls durch Chlor, Fluor, Methyl, Phenyl, Hydroxy, Methoxy, Benzyloxy, Nitro oder Piperidino substituiertes Phenyl, 2-Thienyl oder Hydroxy substituiert sein kann, wobei $R^{12}$ für Wasserstoff, eine verzweigte oder unverzweigte Alkylgruppe mit 1 bis 3 Kohlenstoffatomen, die gegebenenfalls durch eine oder zwei Hydroxygruppen substituiert sein kann, einen Phenacylrest, einen Oxalkylrest mit bis zu 4 Kohlenstoffatomen sowie einen Rest $COR^{13}$ steht, wobei $R^{13}$ Wasserstoff oder Alkyl mit ein oder zwei Kohlenstoffatomen bedeutet,

Z für Sauerstoff, einen Aminrest $NR^{15}$ steht, wobei $R^{15}$ ein Alkylrest mit 1-4 Kohlenstoffatomen oder einen gegebenenfalls durch Halogen, Methyl oder Nitro substituierten Phenylrest darstellt,

und wenn n = 0 bzw. n = 1 für $R^1$-$R^6$ die oben angegebenen Definitionen gelten.

Weiterhin wurde gefunden, daß man die Verbindungen der Formel (I) erhält, wenn man enantiomerenreine Chinoloncarbonsäurederivate der Formel (II)

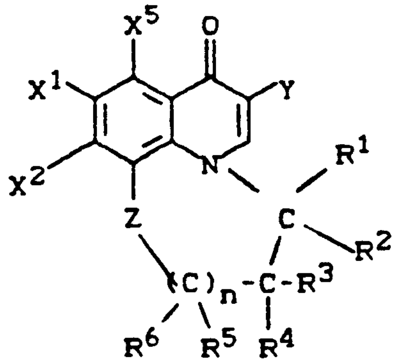

(II)

in der die Reste $X^1$, $X^5$, $R^1$-$R^6$, Z, Y sowie n die oben angegebene Bedeutung haben, sowie $X^2$ für Chlor und Fluor steht,

mit Aminen der Formel (III)

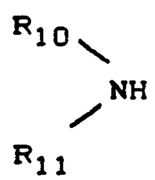

(III)

in welcher

$R_{10}$ und $R_{11}$ die oben angegebene Bedeutung haben,

gegebenenfalls in Gegenwart von Säurebindern umsetzt (Methode A).

Dieses Verfahren muß nicht notwendigerweise so gestaltet werden, daß $R^{10}$ und $R^{11}$ in den Aminen der Formel III bereits ihre endgültige Bedeutung haben, die sie in den erfindungsgemäßen Verbindungen der Formel I haben. Es können vielmehr auch in einem ersten Schritt Vorstufen zu den Resten $R^{10}$ und $R^{11}$ benutzt werden, die dann in einem oder mehreren Folgereaktionsschritten in die endgültige Form von $R^{10}$ und $R^{11}$ überführt werden.

So können beispielsweise erfindungsgemäße Verbindungen der Formel (I) auch erhalten werden, indem man eine enantiomerenreine 10-(1-Piperazinyl)verbindung (bei n=1) der Formel (IV)

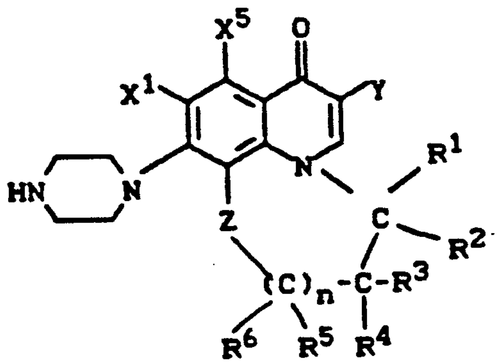

(IV)

**in welcher**

**$X^1$, $X^5$, $R^1$-$R^6$, Z und Y sowie n die oben angegebene Bedeutung haben und**

der Piperazinylrest an den Kohlenstoffatomen in der bei $R^{10}$ und $R^{11}$ angegebenen Weise substituiert sein kann, beispielsweise 1-3 fach durch $C_1$-$C_4$-Alkyl, 2-Thienyl oder gegebenenfalls substituiertes Cyclohexyl oder Phenyl mit Verbindungen der Formel (V)

$$R^{12}X \qquad (V)$$

in welcher

$R^{12}$ die oben angegebene Bedeutunghat, jedoch nicht Wasserstoff sein kann, und

X Fluor, Chlor, Brom, Iod, Hydroxy, Acyloxy, Ethoxy, Phenoxy, 4-Nitrophenoxy bedeutet,

gegebenenfalls in Gegenwart von Säurebindern umsetzt (Methode B).

Bei dieser Reaktionsfürhung kann also der in der 10- bzw. 11-Stellung befindliche Piperazinylrest in einen ersten Reaktionsschritt nach der zuerst genannten Methode eingeführt werden - was zu der bereits erfindungsgemäßen Verbindung IV führt - und in einen Folgeschritt dann gegebenenfalls gewünschte weitere Substituenten eingeführt werden, hier beispielhaft $R^{12}$.

In einer weiteren Ausgestaltung des erfindungsgemäßen Verfahrens erhält man erfindungsgemäße Verbindungen der Formel (I) auch wenn man enantiomerenreine 10-(1-Piperazinyl)chinoloncarbonsäurederivate (n=0) bzw. enantiomerenreine 11-(1-Piperazinyl)chinoloncarbonsäurederivate (n=1) der Formel (IV), in welcher der Piperazinylrest an den Kohlenstoffatomen 1-3 fach durch $C_1$-$C_4$-Alkyl, 2-Thienyl oder gegebenenfalls substituiertes Cyclohexyl oder Phenyl substituiert sein kann, mit Michael-Acceptoren der Formel (VI),

$$B\text{-}CH{=}CH_2 \qquad (VI)$$

in der

B für CN, CO-$R^{22}$ oder COO$R^{23}$ steht, wobei $R^{22}$ für Methyl oder Ethyl und $R^{23}$ für Methyl, Ethyl, n- oder i-Propyl steht, umsetzt (Methode C).

Verwendet man bei der Umsetzung nach Methode A 1-Methylpiperazin und enantiomerenreine 9,10-Difluor-2,3-dihydro-7-oxo-2-phenyl-7H-pyrido [1,2,3-de][1,4]benzoxazin-6-carbonsäure als Ausgangsstoffe,so kann der Reaktionsablauf durch das folgende Formelschema wiedergegeben werden:

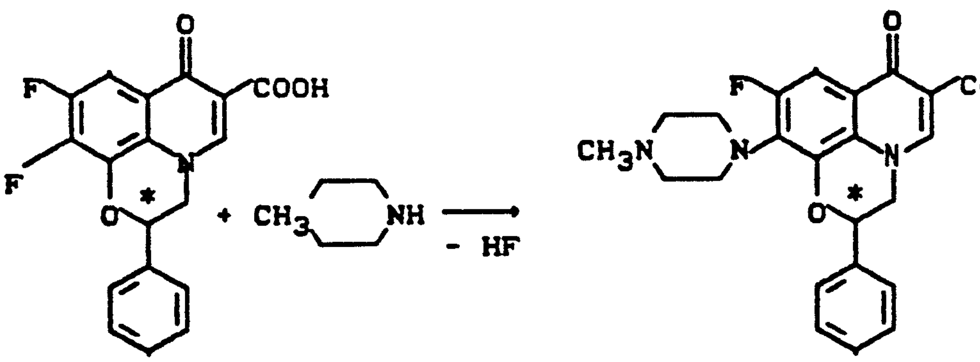


Verwendet man bei der Umsetzung nach Methode B Ethyliodid und enantiomerenreine 9-Fluor-2,3-dihydro-7-oxo-2-phenyl-10(1-piperazinyl)-7H-pyrido[1,2,4-de][1,4]benzoxazin-carbonsäure als Ausgangsstoffe so kann der Reaktionsablauf durch folgendes Formelschema wiedergegeben werden:

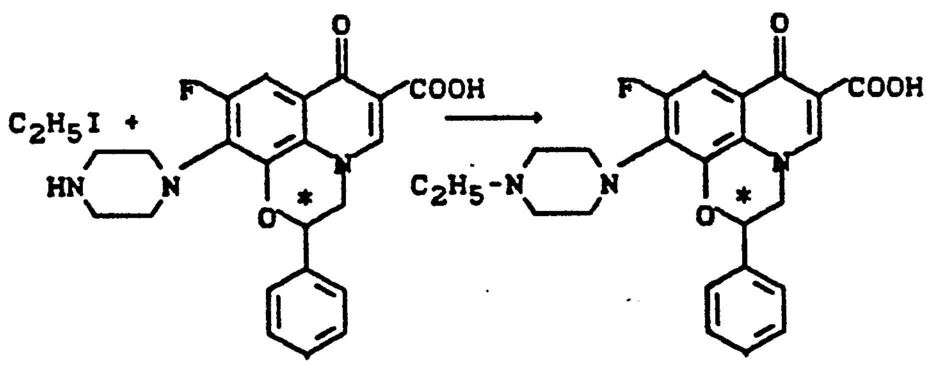

Verwendet man beispielsweise nach Methode C enantiomerenreine 9-Fluor-2,3-dihydro-7-oxo-2-phenyl-10(1-piperazinyl)-7H-pyrido[1,2,3][1,4]benzoxazin-6-carbonsäure und Methylvinylketon als Ausgangsstoffe, so kann der Reaktionsablauf durch folgendes Formelschema wiedergegeben werden:

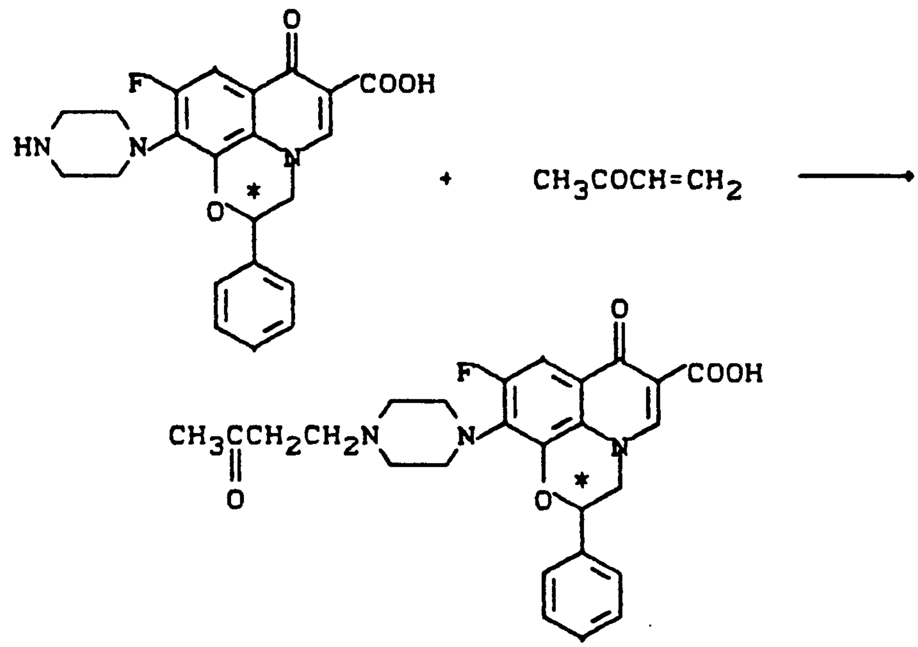

Die als Ausgangsstoffe nach Methode A verwendbaren Chinoloncarbonsäuren der Formel (II) können gemäß folgendem Schema hergestellt werden:

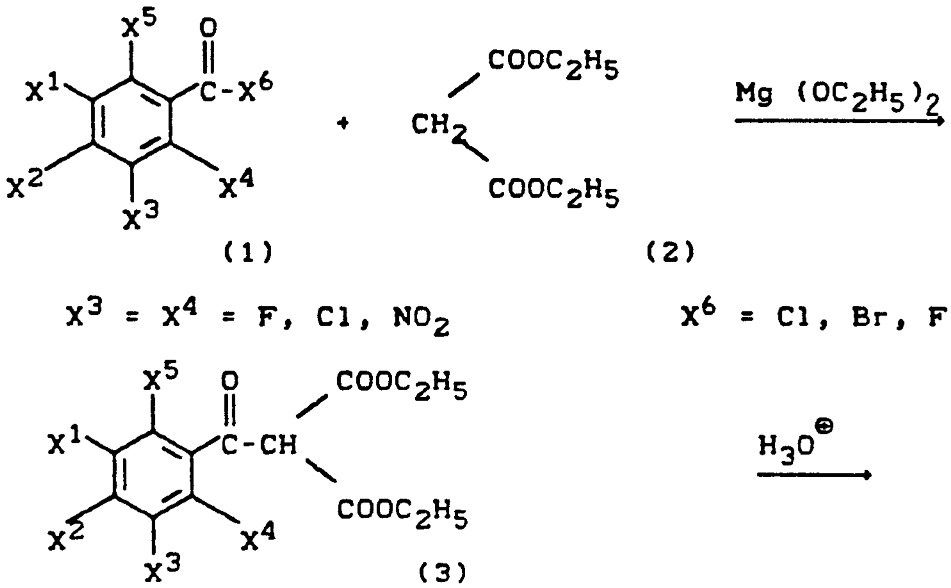


(1) (2)

$X^3 = X^4 = F, Cl, NO_2$ $X^6 = Cl, Br, F$

(3)

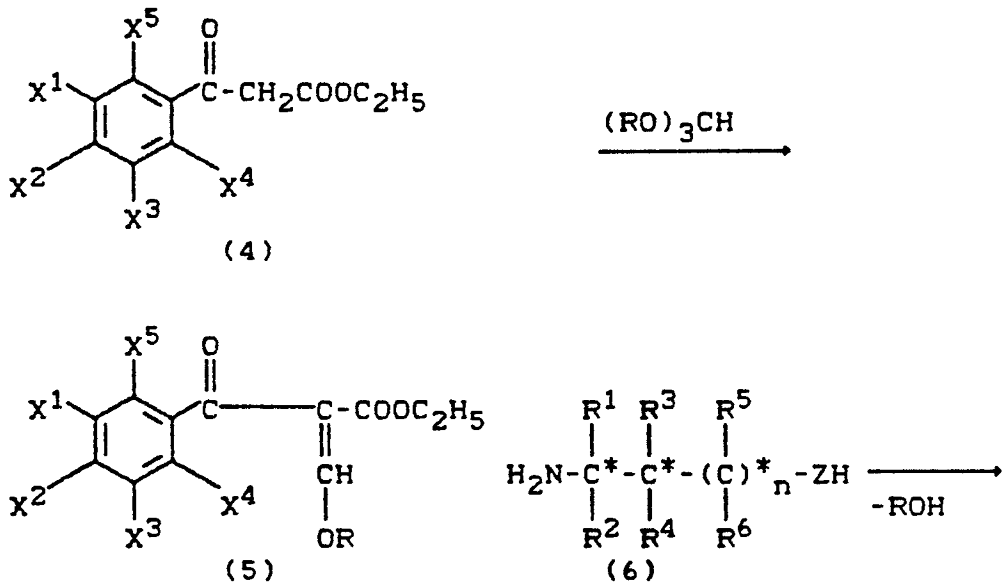


(4)

(5) (6)

R = $CH_3$, $C_2H_5$, $C_3H_7$-n

* gegebenenfalls optisch aktives Zentrum

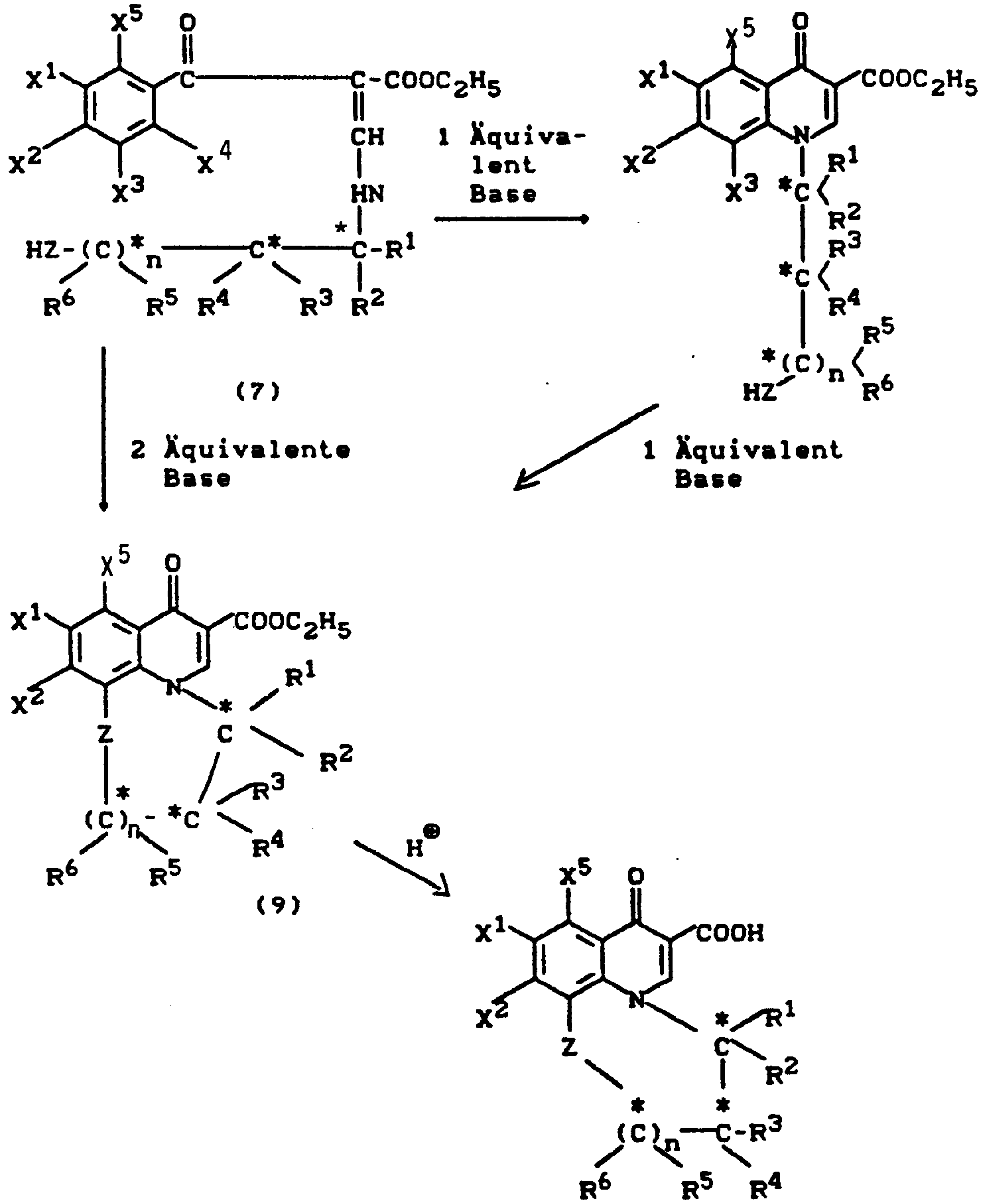


(7)

(9)

* gegebenenfalls optisch aktives Zentrum

Danach wird Malonsäurediethylester (2) in Gegenwart von Magnesiumethylat mit dem entsprechenden Benzoylfluorid bzw. -chlorid (1) zum Benzoylmalonester (3) acyliert (Organicum, 3. Auflage 1964, S. 438).

Durch partielle Verseifung und Decarboxylierung von (3) in wäßrigem Medium mit katalytischen Mengen Schwefelsäure oder p-Toluolsulfonsäure erhält man in guter Ausbeute den Benzoylessigsäureethylester (4), der mit Orthoameisensäuretriethylester/Acetanhydrid in den 3-Ethoxyacrylsäureethylester (5) übergeht. Die Umsetzung von (5) mit dem entsprechenden Amin (6) in einem Lösungsmittel, wie z.B. Methylenchlorid, Alkohol, Chloroform, Cyclohexan oder Toluol führt in leicht exothermer Reaktion zum gewünschten Zwischenprodukt (7).

Die Cyclisierungsreaktionen (7) → (9) bzw. (8) → (9) werden in einem Temperaturbereich von etwa 60 bis 300°C, bevorzugt 80 bis 180°C durchgeführt.

Als Verdünnungsmittel können Dioxan, Dimethylsulfoxid, N-Methylpyrrolidon, Sulfolan, Hexamethylphosphorsäuretriamid und bevorzugt N,N-Dimethylformamid verwendet werden.

Als Säurebinder kommen für diese Reaktionsstufe Kalium-tert.-butanolat, Butyl-lithium, Lithiumphenyl, Phenylmagnesiumbromid, Natriummethylat, Natriumhydrid, Natrium- oder Kaliumcarbonat und besonders bevorzugt Kalium- oder Natrium-fluorid in Betracht.

Für die Primärcyclisierung (7) → (8) und die Zweitcyclisierung (8) → (9) wird im allgemeinen jeweils ein Äquivalent Base verwendet.

Werden beide Cyclisierungsreaktionen in einer "Eintopf-Reaktion" (7) → (9) zusammengefaßt, so müssen 2 Äquivalente der oben angegebenen Basen eingesetzt werden. Es kann vorteilhaft sein, bei den Cyclokondensationen (7) → (9) und (8) → (9) einen Überschuß von ca. 10 Mol-% Base einzusetzen.

Die im letzten Schritt erfolgende Hydrolyse der Ester (9) zu den entsprechenden Carbonsäuren können unter den üblichen bekannten sauren oder basischen Bedingungen erfolgen.

Die als Ausgangsstoffe für diesen Syntheseweg verwendeten 2,3,4,5-Tetrafluorbenzoylchlorid und das Pentafluorbenzoylchlorid sind bekannt.

3,5-Dichlor-2,4-difluor-benzoylfluorid (Siedepunkt 97° / 29 mbar; $n_D^{20}$ = 1,5148) und 5-Chlor-2,3,4-trifluor-benzoylfluorid (Siedepunkt 66-70° /20 mbar; $n_D^{20}$ = 1,4764) erhält man nebeneinander beim Erhitzen von Tetrachlorbenzoylchlorid mit Kaliumfluorid in Sulfolan auf erhöhte Temperaturen:

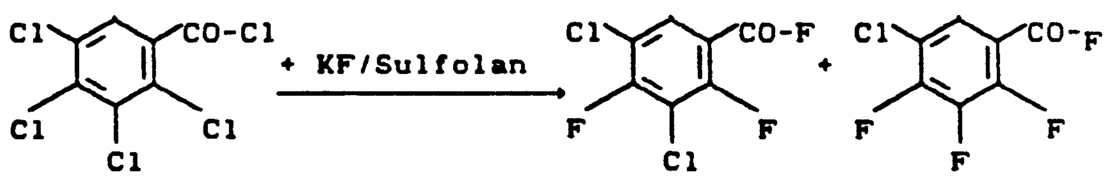

Die Chlorierung von 2,4,5-Trifluorbenzoesäure in Chlorsulfonsäure führt zur 3-Chlor-2,4,5-trifluorbenzoesäure, die als Rohprodukt mit Thionylchlorid zum 3-Chlor-2,4,5-trifluorbenzoylchlorid (Siedepunkt 94° /18 mbar; $n_D^{20}$ = 1,5164) umgesetzt wird:

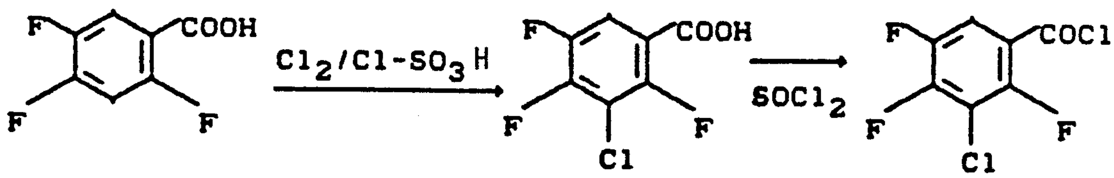


Das 2,4-Dichlor-5-fluor-3-nitro-benzoylchlorid wird durch Nitrierung der bekannten 2,4-Dichlor-5-fluor-benzoesäure zur 2,4-Dichlor-5-fluor-3-nitro-benzoesäure und deren Umsetzung mit Thionylchlorid erhalten.

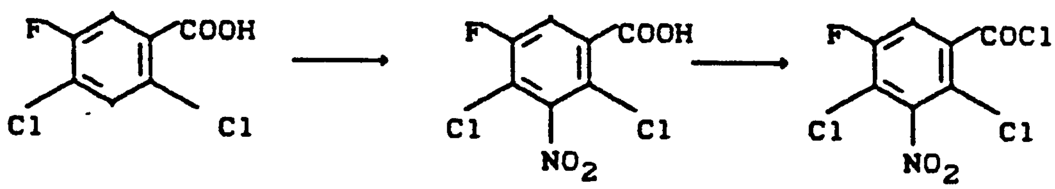

Die als Ausgangsstoffe verwendeten enantiomerenreinen Amine der Formel (6) sind bekannt. Als Beispiele seien genannt:

D-2-Amino-butanol, L-2-Amino-butanol, D-2-Amino-3-methyl-butanol, L-2-Amino-3-methyl-butanol, D-2-Amino-3-phenyl-propanol, L-2-Amino-3-phenyl-propanol, D-2-Amino-2-phenyl-ethanol, L-2-Amino-2-phenyl-ethanol, D-1-Amino-2-propanol, L-1-Amino-2-propanol, D-1,2-Diamino-propan, L-1,2-Diamino-propan, L-1,3-Diamino-butan, D-1,3-Diamino-butan.

Die als Ausgangsstoffe verwendeten Amine (III) sind bekannt oder können nach literaturbekannten Verfahren erhalten werden [US 4 166 180, J. Med. Chem. 26, 1116 (1983)]. Durch katalytische Hydrierung werden aus den 2-Aryl-piperazinen der entsprechenden 2-Cyclohexyl-piperazine erhalten: z.B.: 2-Cyclohexyl-piperazin (wachsartig, Schmelzpunkt 71-73$^0$C). Als Beispiele seien genannt:

Morpholin, Piperidin, Thiomorpholin, Pyrrolidon, Piperazin, N-Methylpiperazin, N-Ethylpiperazin, N-(2-Hydroxyethyl)-piperazin, N-Formylpiperazin, 2-Methylpiperazin, 1,2-Dimethylpiperazin, cis- und trans-2,5-Dimethyl-piperazin, 1-Ethylpiperazin, 2-Propylpiperazin, 2-Isopropylpiperazin, 2-Isobutylpiperazin, 2-Piperazinon, 1-Methyl-2-piperazinon, 1-Ethyl-2-piperazinon, 2-Cyclohexyl-piperazin, 2-Phenylpiperazin, 2-(4-Chlorphenyl)-piperazin, 2-(4-Fluorphenyl)-piperazin, 2-(4-Brom-phenyl)-piperazin, 2-(4-Methylphenyl)-piperazin, 2-(4-Biphenyl)-piperazin, 2-(4-Methoxyphenyl)-piperazin, 2-(4-Benzyloxyphenyl)-piperazin, 2-(4-Hydroxyphenyl)-piperazin, 2-(4-Nitrophenyl)-piperazin, 2-(3-Nitrophenyl)-piperazin, 2-(4-Piperidinophenyl)-piperazin, 2-(3,4-Dimethoxyphenyl)-piperazin, 2-(3,4,5-Trimethoxyphenyl)-piperazin, 2-(3,4-dimethoxy-6-methyl)-piperazin, 2-(2-Thienyl)-piperazin, 3-Aminopyrrolidin.

Die als Ausgangsstoffe verwendeten Verbindungen der Formel (V) sind bekannt. Als Beispiele seien genannt:

Methyliodid, Methylbromid, Ethyliodid, Ethylbromid, Ethylchlorid, 2-Hydroxyethylchlorid, 3-Hydroxypropylchlorid, 4-Hydroxybutylchlorid, n-Propylbromid, i-Propyliodid, n-Butylbromid, i-Butylbromid, sek.-Butylchlorid, n-Pentylchlorid, 3-Methylbutylchlorid, n-Hexylbromid.

Ameisensäureessigsäureanhydrid, Essigsäureanhydrid, Propionsäureanhydrid, Acetylchlorid, Chloracetylchlorid, Dichloracetylchlorid, Bromacetylbromid, Buttersäurechlorid, 4-Chlorbuttersäurechlorid, Isobuttersäurechlorid, N-(tert.-Butoxycarbonyl)-glycin-4-nitrophenylester, N-(tert.-Butoxycarbonyl)-L-alanin-4-nitro-phenylester, N-(tert.-Butoxycarbonyl)-L-leucin-4-nitro-phenylester, N-(tert.-Butoxycarbonyl)-L-valin-4-nitrophenylester, 3-Methoxypropionsäurechlorid, Chlorkohlensäuremethylester, Chlorkohlensäureethylester, Chlorkohlensäure-n-butylester, Diethylcarbonat, Chlorcyan, Diphenylcarbonat, Bromcyan, Dimethylcarbamidsäurechlorid, Methansulfonsäurechlorid, Ethansulfonsäurechlorid, Propan-1-sulfonsäurechlorid, Ameisensäure.

Die erfindungsgmeäß verwendbaren Verbindungen der Formel (VII) sind bekannt. Als Beispiele seien genannt:

Acrylnitril, Methylvinylketon, Acrylsäuremethylester, Acrylsäureethylester.

Die Umsetzung von (II) mit (III) gemäß Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindemittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo-[2,2,2]-octan (DABCO), 1,8-Diazabicyclo[5,4,0]-undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure (II) 1 bis 15 Mol, vorzugsweise 1 bis 6 Mol des Amins (III) ein.

Die Umsetzung von (IV) mit (V) wird vorzugsweise in einem Verdünnungsmittel wie Dimethylsulfoxid, Dioxan, N,N-Dimethylformamid, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindemittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2,2,2]octan (DABCO) oder 1,8-Diazabicyclo[5,4,0]undec-7-en (DBU).

Die Reaktionstemperaturen können in einem größeren Bereich variiert werden. Im allgemeinen arbeitet man zwischen etwa 20 und etwa 180° C, vorzugsweise zwischen 40 und 110° C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 4 Mol, vorzugsweise 1 bis 1,5 Mol, der Verbindung (V) ein.

Die Umsetzung von (IV) mit (VI) (Methode C) wird vorzugsweise in einem Verdünnungsmittel wie Dioxan, Dimethylsulfoxid, N,N-Dimethylformamid, Methanol, Ethanol, Isopropanol, n-Propanol, Glykolmonomethylether oder auch in Gemischen dieser Verdünnungsmittel durchgeführt.

Die Reaktionstemperaturen können in einem größeren Bereich variiert werden. Im allgemeinen arbeitet man zwischen etwa 20° C und etwa 150° C, vorzugsweise zwischen 50° C und 100° C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens nach Methode C setzt man auf 1 Mol der Verbindung (IV) 1 bis 5 Mol, vorzugsweise 1 bis 2 Mol, der Verbindung (VI) ein.

Außerdem den in den Beispielen aufgeführten Verbindungen seien als neue Wirkstoffe im einzelnen genannt:

2R-9-Fluor-2-fluormethyl-2,3-dihydro-7-oxo-10(1-pyrrolidinyl)-7H-pyrido[1,2,3]benzoxazin-6-carbonsäure,
2R-9-Chlor-2,3-dihydro-2-hydroxymethyl-10(4-methyl-1-piperazinyl)-7-oxo-7H-pyrido[1,2,3-de][1,4]benzoxazin-6-carbonsäure,
2R-9-Fluor-2,3-dihydro-2-hydroxymethyl-10(4-methyl-1-piperazinyl)-7-oxo-7H-pyrido[1,2,3-de][1,4]-benzoxazin-6-carbonsäure,

2S-9-Fluor-2,3-dihydro-7-oxo-2-phenyl-10-(1-pyrrolidinyl)-7H-pyrido[1,2,3-de][1,4]-benzoxazin-6-carbonsäure,

2S-9-Chlor-2,3-dihydro-7-oxo-2-phenyl-10-(1-pyrrolidinyl)-7H-pyrido[1,2,3-de][1,4]-benzoxazin-6-carbonsäure,

3S-9-Fluor-2,3-dihydro-2,2-dimethyl-3-methyl-10-(4-methyl-1-piperazinyl)-7-oxo-7H-pyrido[1,2,3-de][1,4]-benzoxazin-6-carbonsäure,

2R-9-Fluor-2,3-dihydro-2-methyl-3,3-dimethyl-10-(4-methyl-1-piperazinyl)-7-oxo-7H-pyrido[1,2,3-de][1,4]-benzoxazin-6-carbonsäure,

3aR, 11aS-6-Fluor-3a,11a-dihydro-8-oxo-5-(1-pyrrolidinyl)-8H-cyclopenta[1,2-b]-pyrido[1,2,3-de][1,4]-benzoxazin-9-carbonsäure,

3aR, 11aS-6-Chlor-3a,11a-dihydro-8-oxo-5(1-pyrrolidinyl)-8H-cyclopenta[1,2-b]-pyrido[1,2,3-de][1,4]-benzoxazin-9-carbonsäure,

3aR, 11aS-6-Fluor-3a, 11a-dihydro-8-oxo-5(3-phenyl-1-piperazinyl)-8H-cyclopenta[1,2-b]-pyrido[1,2,3-de][1,4]-benzoxazin-9-carbonsäure,

3S-9-Fluor-2,3-dihydro-3-methyl-10(4-methyl-1-piperazinyl)-7-oxo-1H, 7H-pyrido[1,2,3-de]-chinoxalin-6-carbonsäure,

3S-9-Fluor-2,3-dihydro-3-methyl-7-oxo-10(-1-piperazinyl)-1H,7H-pyrido[1,2,3-de]-chinoxalin-6-carbonsäure,

3S-9-Fluor-2,3-dihydro-1,3-dimethyl-10(4-methyl-1-piperazinyl)-7-oxo-7H-pyrido[1,2,3-de]-chinoxalin-6-carbonsäure,

3S-9-Fluor-2,3-dihydro-1,3-dimethyl-10(3-methyl-1-piperazinyl)-7-oxo-7H-pyrido[1,2,3-de]-chinoxalin-6-carbonsäure,

2R-9-Fluor-2,3-dihydro-1,2-dimethyl-10(4-methyl-1-piperazinyl)-7-oxo-7H-pyrido[1,2,3-de]-chinoxalin-6-carbonsäure,

2R-9-Fluor-2,3-dihydro-3-methyl-10(4-methyl-1-piperazinyl)-7-oxo-2-phenyl-7H-pyrido[1,2,3-de]-chinoxalin-6-carbonsäure,

4S-10-Fluor-2,3-dihydro-4-methyl-11(4-methyl-1-piperazinyl)-8-oxo-4H, 8H-pyrido[1,2,3-ef][1,5]-benzoxazepin-7-carbonsäure,

4S-10-Fluor-2,3-dihydro-4-methyl-11(3-methyl-1-piperazinyl)-8-oxo-4H, 8H-pyrido[1,2,3-ef][1,5]-benzoxazepin-7-carbonsäure

4S-10-Fluor-2,3-dihydro-4-methyl-8-oxo-11(1-pyrrolidinyl)-4H, 8H-pyrido[1,2,3-ef][1,5]-benzoxazepin-7-carbonsäure,

3R-10-Chlor-2,3-dihydro-3-methyl-11(4-methyl-1-piperazinyl)-8-oxo-4H, 8H-pyrido[1,2,3-ef][1,5]-benzoxazepin-7-carbonsäure,

4aR, 12aS-7-fluor-4-a, 12a-dihydro-9-oxo-6(1-piperazinyl)-9H-cyclohexa[1,2-b]-pyrido[1,2,3-de][1,4]-benzoxazin-10-carbonsäure,

4aR, 12aS-7-Chlor-4a, 12a-dihydro-9-oxo-6(1-pyrrrolidinyl)-9H-cyclohexa[1,2-b]-pyrido[1,2,3-de][1,4]-benzoxazin-10-carbonsäure,

4aR, 12aS-7-Fluor-4a, 12a-dihydro-6-(4-methyl-1-piperazinyl)-9-oxo-9H-cyclohexa-[1,2-b]-pyrido[1,2,3-de][1,4]-benzoxazin-10-carbonsäure.

## Beispiel für eine erfindungsgemäße Tablette

Jede Tablette enthält:

| | |
|---|---|
| Verbindung des Beispiels 4 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Macrogol 4000 rec. INN Polyethylenglykol (DAB) | 2,0 mg |
| Titan-(IV)-oxid | 2,0 mg |
| | 10,0 mg |

Die erfindungsgemäßen enantiomerenreinen Verbindungen zeigen in den üblichen Konzentrationen bessere Wirkungen als die bei gängigen Herstellungsmethoden für 1,8-verbrückte Chinoloncarbonsäuren anfallenden Racemate.

Durch geringere Aufwandmengen und die Abwesenheit unwirksamer Enantiomerer sind die erfindungsgemäßen Verbindungen herkömmlichen 1,8-verbrückten Chinoloncarbonsäuren, die als Racemate vorliegen, weit überlegen.

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die

folgenden Erreger oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Metritis-Mastitis-Agalaktiae-Syndrom, Mastitis;

Wiederkäuer (Rind, Schaf, Ziege): Diarrhoe, Sepsis, Bronchopneumonie, Salmonellose, Pasteurellose, Mykoplasmose, Genitalinfektionen;

Pferd: Bronchopneumonien, Fohlenlähme, puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die nachstehende Tabelle zeigt einen Vergleich der MHK-Werte von 3S-9-Fluor-2,3-dihydro-3-methyl-10(4-methyl-1-piperazinyl)-7-oxo-7H-pyrido[1,2,3-de][1,4]-benzoxazin-6-carbonsäure mit dem entsprechenden Racemat (Ofloxacin).

MHK (mcg/ml)

| E. coli | Beispiel 4 | Beispiel 16 | Ofloxacin |
|---|---|---|---|
| 4418 | 0,125 | 2 | 0,25 |
| T 7 | 0,03 | 1 | 0,06 |
| A 261 | ≤ 0,015 | 0,25 | 0,03 |
| **Klebsiella** | | | |
| 63 | 0,06 | 2 | 0,125 |
| 57 USA | 0,125 | 4 | 0,25 |
| 6318 | 0,125 | 4 | 0,25 |
| **Proteus** | | | |
| 8175 | 0,06 | 4 | 0,125 |
| Vulg. 1017 | 0,06 | 2 | 0,125 |
| **Providencia** | | | |
| 12012 | 0,06 | 4 | 0,25 |
| **Staph.** | | | |
| F 422 | 0,25 | 16 | 0,5 |
| 1756 | 0,25 | 8 | 0,5 |
| 133 | 0,25 | 8 | 0,5 |
| **Strepto** | | | |
| 9790 | 1 | 32 | 2 |

| | Beispiel 4 | Beispiel 16 | Ofloxacin |
|---|---|---|---|
| Pseudomonas | | | |
| Walter | 1 | 32 | 2 |
| Ellsworth | 0,25 | 4 | 0,5 |

Agar dilutionstest (Isosensitest-Medium); Denley Multipointinoculator

Die folgenden Beispiele erläutern die Erfindung:

Beispiel 1

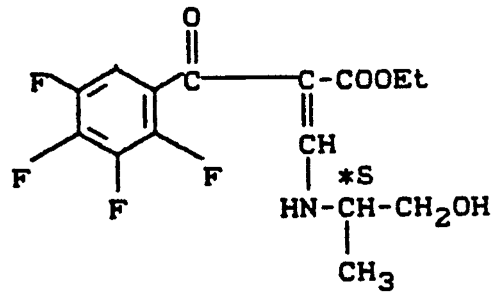

8 g 3-Ethoxy-2-(2,3,4,5-tetrafluorbenzoyl)-acrylsäureethylester werden in 10 ml Ethanol vorgelegt. Unter Eiskühlung werden 2,1 g S-(+)-2-Aminopropanol in 10 ml EtOH zugetropft. Man läßt 2 Stunden bei RT rühren und engt dann im Vakuum ein. Es bleiben 9,5 g (Roh) der Titelverbindung zurück.

Beispiel 2

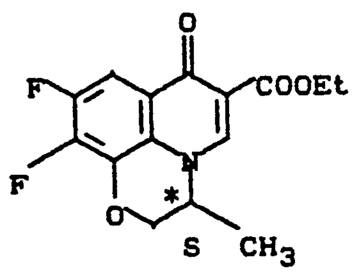

9,5 g des Rohproduktes aus Beispiel 1 werden mit 4,3 g $K_2CO_3$ in 40 ml DMF 4 Stunden auf 140°C erhitzt. Nach Abkühlen auf Raumtemperatur gibt man Wasser zu und isoliert den ausgefallenen Feststoff. Ausbeute: 5,7 g 3S-9,10-Difluor-3-methyl-7-oxo-7H-pyrido[1,2,3-de][1,4]-benzoxazin-6-carbonsäureethylester.
Schmelzpunkt: 238-42°C.

**Beispiel 3**

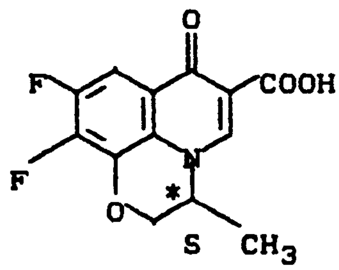

5,7 g Produkt aus Beispiel 2 werden zusammen mit 21 ml Essigsäure, 16 ml Wasser und 1,8 ml Schwefelsäure 4 Stunden auf 150° C (Bad) erhitzt. Nach Abkühlen und Wasserbeigabe werden 4,6 g 3S-9,10-Difluor-3-methyl-7-oxo-7H-pyrido[1,2,3-de][1,4]-benzoxazin-6-carbonsäure erhalten. Schmelzpunkt: > 300° C.

**Beispiel 4**

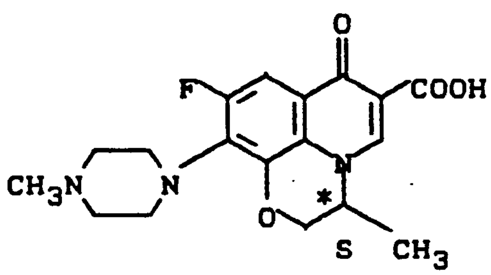

4,6 g Produkt aus Beispiel 3 und 5,1 g N-Methyl-piperazin werden in 50 ml DMSO 2,5 Stunden auf 140° C erhitzt. Danach wird das DMSO im Hochvakuum abgezogen. Den verbleibenden Rückstand kocht man mit EtOH auf und isoliert den ausgefallenen Feststoff. Ausbeute: 2,0 g 3S-9-Fluor-3-methyl-10(4-methyl-1-piperazinyl)-7-oxo-7H-pyrido[1,2,3-de][1,4]-benzoxazin-6-carbonsäure.

0225552

Nach Umkristallisation aus DMF hat die Verbindung einen Schmelzpunkt von 298-300°C (Z).

$[\alpha]_D^{20}$ = -55,5° (C = 0,18 1n HCl)

Beispiel 5

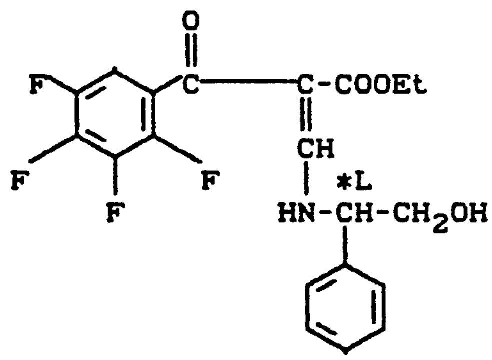

6,4 g 3-Ethoxy-2-(2,3,4,5-tetrafluorbenzoyl)-acryl-säure-ethylester werden in 8 ml Ethanol vorgelegt. Unter Kühlung wird eine Lösung von 3 g L(+) α-Phenylglycinol in 15 ml Ethanol zugetropft. Man läßt 3 Stunden bei Raumtemperatur rühren und engt dann ein.
Ausbeute: 9,2 g der Titelverbindung als Öl.

Beispiel 6

Analog zu Beispiel 5 wird der Ethoxyacrylester mit D(-)α-Phenylglycinol umgesetzt.
Ausbeute: 9,2 g Öl.

Beispiel 7

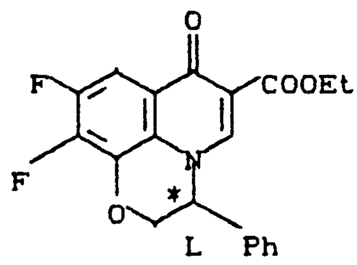

9,2 g Produkt aus Beispiel 5 werden mit 3,3 g Kaliumcarbonat in 40 ml DMF 4 Stunden auf 140°C erhitzt. Nach Abkühlung auf Raumtemperatur wird mit Wasser verdünnt und der Feststoff isoliert. Nach dem Trocknen wird mit wenig Methanol verrührt.

Ausbeute: 5 g 3L-9,10-Difluor-3-phenyl-7-oxo-7H-pyrido-[1,2,3-de][1,4]-benzoxazin-6-carbonsäureethylester
Schmelzpunkt: > 300°C.

Beispiel 8

Analog Beispiel 7 werden aus 9,4 g Produkt Beispiel 6 3,3 g 3D-9,10-Difluor-3-phenyl-7-oxo-7H-pyrido-[1,2,3-de][1,4]-benzoxazin-6-carbonsäureethylester erhalten.

Schmelzpunkt: > 300°C.

Beispiel 9

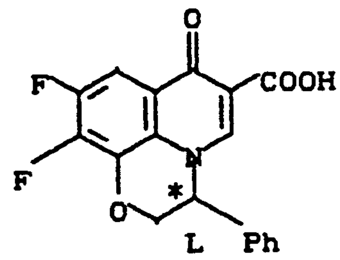

4 g Produkt aus Beispiel 7 werden zusammen mit 13 ml Essigsäure, 12 ml Wasser und 1,2 ml Schwefelsäure 6 Stunden gekocht. Danach gibt man Wasser zu und isoliert den ausgefallenen Feststoff.
Ausbeute: 3,3 g 3L-9,10-Difluor-3-phenyl-7-oxo-7H-pyrido-[1,2,3-de][1,4]-benzoxazin-6-carbonsäure
Schmelzpunkt: 181-82° C.

Beispiel 10

Analog zu Beispiel 9 werden aus 2,2 g Vorprodukt Beispiel 8 2,0 g 3D-9,10-Difluor-3-Phenyl-7-oxo-7H-pyrido-[1,2,3-de][1,4]-benzoxazin-6-carbonsäure erhalten.

Beispiel 11

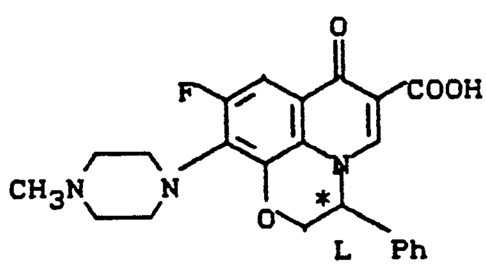

2,8 g Produkt aus Beispiel 9 und 4 g N-Methyl-piperazin werden in 25 ml DMSO 2,5 Stunden auf 140° C erhitzt. Danach wird alles Flüchtige im Hochvakuum abdestilliert.

Den Rückstand verrührt man mit Wasser und isoliert den anfallenden Feststoff.

Ausbeute: 2,6 g 3L-9-Fluor-10(4-methyl-1-piperazinyl)-3-phenyl-7-oxo-7H-pyrido[1,2,3-de][1,4]-benzoxazin-6-carbonsäure.
Schmelzpunkt: 252-3° (Z).

Beispiel 12

Analog zu Beispiel 11 werden aus 1,5 g Vorprodukt Beispiel 10 1,4 g 3D-9-Fluor-10(4-methyl-1-piperazinyl)-3-phenyl-7-oxo-7H-pyrido[1,2,3-de][1,4]-benzoxazin-6-carbonsäure erhalten.
Schmelzpunkt 257° C (Z).

Beispiel 13

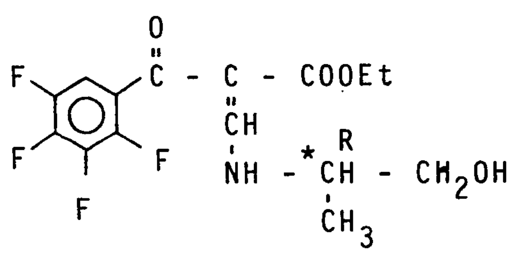

5,76 g 3-Ethoxy-2-(2,3,4,5-tetrafluorbenzoyl)-acrylsäureethylester werden in 10 ml Ethanol vorgelegt. Unter Eiskühlung werden 1,5 g R-(-)-2-Amino-propanol in 10 ml Ethanol zugetropft. Man läßt 2 Stunden bei RT rühren und engt dann im Vakuum ein. Es bleiben 6,8 g (roh) der Titelverbindung zurück.

Beispiel 14

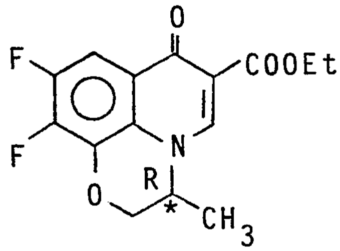

6,8 g des Rohproduktes aus Beispiel 13 werden mit 3 g Kaliumcarbonat in 30 ml DMF 4 Stunden auf 140°C erhitzt. Nach Abkühlen auf RT gibt man Wasser zu und isoliert den ausgefallenen Feststoff.

Ausbeute: 4,2 g 3R-9,10-Difluor-3-methyl-7-oxo-7H-pyrido-/1,2,3-de7/1,47-benzoxazin-6-carbonsäureethylester.

Schmelzpunkt: 236-238°C.

Beispiel 15

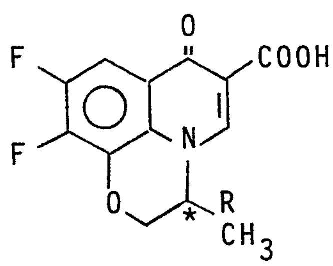

4,0 g Produkt aus Beispiel 14 werden zusammen mit 15 ml Essigsäure, 11 ml Wasser und 1,3 ml Schwefelsäure 4 Stunden auf 140°C erhitzt. Nach Abkühlen und Wasserzugabe werden 3,5 g 3R-9,10-Difluor-3-methyl-7-oxo-7H-pyrido-/1,2,3-de7/1,47-benzoxazin-6-carbonsäure erhalten.

Schmelzpunkt: >300°C

Beispiel 16

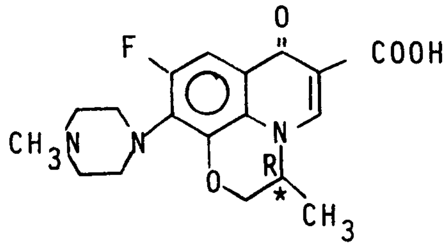

3,5 g Produkt aus Beispiel 15 und 3,9 g N-Methylpiperazin werden in 30 ml DMSO 2,5 Stunden auf 140°C erhitzt. Danach wird alles Flüchtige im Hochvakuum abgezogen. Der verbleibende Rückstand wird mit EtOH versetzt. Man isoliert den ausgefallenen Feststoff und kristallisiert aus DMF um.

Ausbeute: 2,0 g 3R-9-Fluor-3-methyl-10(4-methyl-1-piperazinyl)-7-oxo-7H-pyrido/1,2,3-de/ /1,4/-benzoxazin-6-carbonsäure.

Schmelzpunkt: 295-298°C (Z)

$[\alpha]_D^{20}$ = + 53,8° (C=0,18 1n HCl).

Patentansprüche

1. Enantiomerenreine 1,8-verbrückte 4-Chinolon-3-carbonsäuren und Derivaten der Formel (I)

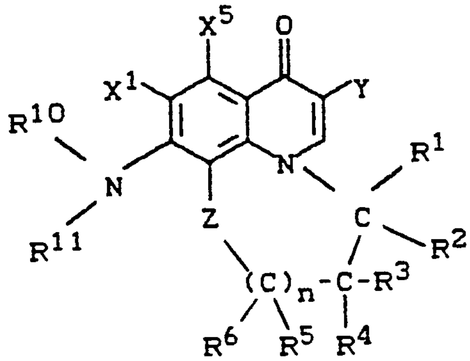

(I)

in der

Y eine Carboxylgruppe, eine Nitrilgruppe, eine Estergruppe $-COOR^7$ oder eine Säureamidgruppe $-CONR^8R^9$ darstellt, wobei $R^7$ für $C_1$-$C_4$-Alkyl steht, $R^8$ und $R^9$ für Wasserstoff oder $C_1$-$C_4$-Alkyl stehen, $R^9$ außerdem gegebenenfalls substituiertes Phenyl sein kann,

$X^1$ für Wasserstoff, Nitro, Alkyl mit 1 bis 3 Kohlenstoffatomen, Halogen, bevorzugt Fluor steht,

$X^5$ Wasserstoff, Halogen oder Methyl sein kann,

$R^{10}$ und $R^{11}$ gemeinsam mit dem Stickstoffatom, an das sie gebunden sind, einen 5- oder 6-gliedrigen heterocyclischen Ring bilden, der als Ringglied zusätzlich

die Atome oder Gruppen -O-, -S-, -SO-, $-SO_2-$,

$\rangle N-R^{12}$

oder $-CO-\overset{|}{N}-R^{12}$ enthalten kann und der gegebenenfalls an den Kohlenstoffatomen ein- bis dreifach durch $C_1$-$C_4$-Alkyl, gegebenenfalls durch Chlor, Fluor, Brom, Methyl, Phenyl, Hydroxy, Methoxy, Benzyloxy, Nitro oder Piperidino ein- bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und Ethylaminomethyl substituiert sein kann, wobei

$R^{12}$ für Wasserstoff, eine verzweigte oder unverzweigte Alkyl-, Alkenyl- oder Alkinylgruppe mit 1 bis 6 Kohlenstoffatomen, die gegebenenfalls durch ein oder zwei Hydroxy-, Alkoxy-, Alkylamino- oder Dialkylaminogruppen mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, eine gegebenenfalls in Phenylrest substituierte Phenylalkylgruppe mit bis zu 4 Kohlenstoffatomen im aliphatischen Teil,

einen gegebenenfalls durch Hydroxy, Methoxy, Chlor oder Fluor ein- oder zweifach substituierten Phenacylrest, einen Oxoalkylrest mit bis zu 6 Kohlenstoffatomen steht, ferner einen Rest $COR^{13}$ oder $SO_2R^{14}$ bedeutet, wobei

$R^{13}$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Alkoxy mit 1 bis 4 Kohlenstoffatomen, Amino, Alkylamino oder Dialkylamino mit 1 bis 5 Kohlenstoffatomen im Alkylteil und

$R^{14}$ geradkettiges oder verzweigtes Alkyl mit 1 bis 3 Kohlenstoffatomen darstellt, und

Z für Sauerstoff, einen Aminrest $NR^{15}$ steht, wobei $R^{15}$ Wasserstoff, einen gegebenenfalls durch Halogen, Trifluormethyl, Nitro, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen, einen gegebenenfalls durch Halogen, eine Nitrogruppe, oder Alkylgruppe mit 1-3 Kohlenstoffatomen, eine Alkoxy- bzw. Alkylmercaptogruppe mit jeweils 1-3 Kohlenstoffatomen substituierten Phenylrest bedeutet, sowie ferner einen Acylrest $R^{16}$-CO-bzw.

$R^{17}SO_2$- darstellt, wobei $R^{16}$ bzw. $R^{17}$ Alkylreste mit 1-6 Kohlenstoffatomen oder gegebenenfalls substituierte Phenylreste darstellen, sowie ein

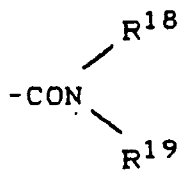

oder

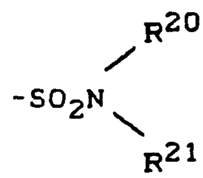

-Rest sein kann, wobei die Reste $R^{18}$ bis $R^{21}$ Wasserstoff, Alkyl mit 1-6 Kohlenstoffatomen oder einen gegebenenfalls substituierten Phenylrest darstellen

und

$R^1$, $R^2$, $R^3$, $R^4$, $R^5$ und $R^6$ für Wasserstoff, eine gegebenenfalls ein-oder mehrfach durch Halogen, insbesondere Chlor oder Fluor, ferner Nitro, Cyano, Hydroxy, Alkoxy, bzw. Alkylmercapto mit 1-3 Kohlenstoffatomen im Alkoholteil substituierte Alkylgruppe mit 1-6 Kohlenstoffatomen, einen gegebenenfalls durch Halogen, Nitro, Alkyl bzw. Alkoxy bzw. Alkylmercapto mit jeweils bis zu 3 Kohlenstoffatomen, Hydroxy, Aryloxy, Arylthio, Cyano oder einen Esterrest mit 1-3 Kohlenstoffatomen im Alkoholteil substituierten Phenylrest, Naphthylrest oder heterocyclischen Rest wie z.B. Thiophen-, Furan-, Pyrrol-, Thiazol-, Pyridin- oder Pyrimidin-Rest stehen mit der Maßgabe, daß

$R^1$ und $R^2$ und/oder $R^3$ und $R^4$ und/oder $R^5$ und $R^6$ verschieden sind, sowie $R^2$ mit $R^3$ und/oder $R^4$ mit $R^5$ jeweils mit dem Kohlenstoffatomen, an das sie gebunden sind, einen 3-7 gliedrigen Ring bilden, der gegebenenfalls durch gegebenenfalls substituierte Alkylreste mit 1-3 Kohlenstoffatomen oder gegebenenfalls substituierte Arylreste substituiert ist und

n = 0 oder 1 bedeutet,

und deren pharmazeutisch verwendbare Hydrate, Alkali-, Erdalkali-, Silber- und Guanidiniumsalze, sowie ihre Ester.

2. Enantiomerenreine 1,8-verbrückte 4-Chinolon-3-carbonsäuren der Formel (I) gemäß Anspruch 1, in der

Y eine Carboxylgruppe, eine Nitrilgruppe, eine Estergruppe $-COOR^7$ darstellt, wobei $R^7$ Methyl oder Ethyl sein kann,

$X^1$ für Fluor steht,

$X^5$ für Wasserstoff steht,

$R^{10}$ und $R^{11}$ gemeinsam mit dem Stickstoffatom, an das sie gebunden sind, einen 5- oder 6-gliedrigen heterocyclischen Ring bilden, der als Ringglied zusätzlich ein Sauerstoffatom oder die Gruppen

$>N-R^{12}$

oder $-CO-N-R^{12}$ enthalten kann und der gegebenenfalls an den Kohlenstoffatomen ein- bis dreifach durch $C_1-C_2$-Alkyl, Cyclohexyl, gegebenenfalls durch Chlor, Fluor, Methyl, Phenyl, Hydroxy, Methoxy, Benzoyloxy, Nitro doer Piperidino substituiertes Phenyl, 2-Thienyl oder Hydroxy substituiert sein kann, wobei $R^{12}$ für Wasserstoff, eine verzweigte oder unverzweigte Alkylgruppe mit 1 bis 3 Kohlenstoffatomen, die gegebenenfalls durch eine oder zwei Hydroxygruppen substituiert sein kann, einen Phenacrylrest, einen Oxoalkylrest mit bis zu 4 Kohlenstoffatomen sowie einen Rest $COR^{13}$ steht, wobei $R^{13}$ Wasserstoff oder Alkyl mit ein oder zwei Kohlenstoffatomen bedeutet,

Z für Sauerstoff, einen Aminrest $NR^{15}$ steht, wobei $R^{15}$ ein Alkylrest mit 1-4 Kohlenstoffatomen oder einen gegebenenfalls durch Halogen, Methyl oder Nitro substituierten Phenylrest darstellt,

und in der bei n=o bzw. n=1 für $R^1$ bis $R^6$ die in Anspruch 1 angegebenen Definitionen gelten.

3. Enantiomerenreine 1,8-verbrückte 4-Chinolon-3-carbonsäuren aus der Gruppe bestehend aus

2R-9-Fluor-2-fluormethyl-2,3-dihydro-7-oxo-10(1-pyrrolidinyl)-7H-pyrido[1,2,3-de][1,4]-benzoxazin-6-carbonsäure,

2R-9-Chlor-2,3-dihydro-2-hydroxymethyl-10(4-methyl-1-piperazinyl)-7-oxo-7H-pyrido[1,2,3-de][1,4]benzoxazin-6-carbonsäure,

2R-9-Fluor-2,3-dihydro-2-hydroxymethyl-10(4-methyl-1-piperazinyl)-7-oxo-7H-pyrido[1,2,3][1,4]-benzoxazin-6-carbonsäure,

2S-9-Fluor-2,3-dihydro-7-oxo-2-phenyl-10-(1-pyrrolidinyl)-7H-pyrido[1,2,3-de][1,4]-benzoxazin-6-carbonsäure,

2S-9-Chlor-2,3-dihydro-7-oxo-2-phenyl-10-(1-pyrrolidinyl)-7H-pyrido[1,2,3-de][1,4]-benzoxazin-6-carbonsäure,

3S-9-Fluor-2,3-dihydro-2,2-dimethyl-3-methyl-10-(4-methyl-1-piperazinyl)-7-oxo-7H-pyrido[1,2,3-de][1,4]-benzoxazin-6-carbonsäure,

2R-9-Fluor-2,3-dihydro-2-methyl-3,3-dimethyl-10-(4-methyl-1-piperazinyl)-7-oxo-7H-pyrido[1,2,3-de][1,4]-benzoxazin-6-carbonsäure,

3aR, 11aS-6-Fluor-3a,11a-dihydro-8-oxo-5-(1-pyrrolidinyl)-8H-cyclopenta[1,2-b]-pyrido[1,2,3-de][1,4]-benzoxazin-9-carbonsäure,

3aR, 11aS-6-Chlor-3a,11a-dihydro-8-oxo-5(1-pyrrolidinyl)-8H-cyclopenta[1,2-b]-pyrido[1,2,3-de][1,4]-benzoxazin-9-carbonsäure,

3aR, 11aS-6-Fluor-3a, 11a-dihydro-8-oxo-5(3-phenyl-1-piperazinyl)-8H-cyclopenta[1,2-b]-pyrido[1,2,3-de][1,4]-benzoxazin-9-carbonsäure,

3S-9-Fluor-2,3-dihydro-3-methyl-10(4-methyl-1-piperazinyl)-7-oxo-1H, 7H-pyrido[1,2,3-de]-chinoxalin-6-carbonsäure,

3S-9-Fluor-2,3-dihydro-3-methyl-7-oxo-10(-1-piperazinyl)-1H,7H-pyrido[1,2,3-de]-chinoxalin-6-carbonsäure,

3S-9-Fluor-2,3-dihydro-1,3-dimethyl-10(4-methyl-1-piperazinyl)-7-oxo-7H-pyrido[1,2,3-de]-chinoxalin-6-carbonsäure,

3S-9-fluor-2,3-dihydro-1,3-dimethyl-10(3-methyl-1-piperazinyl)-7-oxo-7H-pyrido[1,2,3-de]-chinoxalin-6-carbonsäure,

2R-9-Fluor-2,3-dihydro-1,2-dimethyl-10(4-methyl-1-piperazinyl)-7-oxo-7H-pyrido[1,2,3-de]-chinoxalin-6-carbonsäure,

2R-9-Fluor-2,3-dihydro-3-methyl-10(4-methyl-1-piperazinyl)-7-oxo-2-phenyl-7H-pyrido[1,2,3-de]-chinoxalin-6-carbonsäure,

4S-10-Fluor-2,3-dihydro-4-methyl-11(4-methyl-1-piperazinyl)-8-oxo-4H, 8H-pyrido[1,2,3-ef][1,5]-benzoxazepin-7-carbonsäure,

4S-10-Fluor-2,3-dihydro-4-methyl-11(3-methyl-1-piperazinyl)-8-oxo-4H, 8H-pyrido[1,2,3-ef][1,5]-benzoxazepin-7-carbonsäure

4S-10-Fluor-2,3-dihydro-4-methyl-8-oxo-11(1-pyrrolidinyl)-4H, 8H-pyrido[1,2,3-ef][1,5]-benzoxazepin-7-carbonsäure,

3R-10-Chlor-2,3-dihydro-3-methyl-11(4-methyl-1-piperazinyl)-8-oxo-4H, 8H-pyrido[1,2,3-ef][1,5]-benzoxazepin-7-carbonsäure,

4aR, 12aS-7-Fluor-4-a, 12a-dihydro-9-oxo-6(1-piperazinyl)-9H-cyclohexa[1,2-b]-pyrido[1,2,3-de][1,4]-benzoxazin-10-carbonsäure,

4aR, 12aS-7-Chlor-4a, 12a-dihydro-9-oxo-6(1-pyrrrolidinyl)-9H-cyclohexa[1,2-b]-pyrido[1,2,3-de][1,4]-benzoxazin-10-carbonsäure,

4aR, 12aS-7-Fluor-4a, 12a-dihydro-6-(4-methyl-1-piperazinyl)-9-oxo-9H-cyclohexa-[1,2-b]-pyrido[1,2,3-de][1,4]-benzoxazin-10-carbonsäure.

4. Enantiomerenreine 1,8-verbrückte 4-Chinolon-3-carbonsäuren bzw. -ester aus der Gruppe bestehend aus

3S-9,10-Difluor-3-methyl-7-oxo-7H-pyrido[1,2,3-de]-[1,4]-benzoxacin-6-carbonsäureethylester;

3S-9,10-Difluor-3-methyl-7-oxo-7H-pyrido[1,2,3-de]-[1,4]-benzoxazin-6-carbonsäure;

3S-9-Fluor-3-methyl-10(4-methyl-1-piperazinyl)-7-oxo-7H-pyrido[1,2,3-de][1,4]-benzoxazin-6-carbonsäure,

3L-9,10-Difluor-3-phenyl-7-oxo-7H-pyrido-[1,2,3-de]-[1,4]-benzoxazin-6-carbonsäureethylestr;

3D-9,10-Difluor-3-phenyl-7-oxo-7H-pyrido-[1,2,3-de]-[1,4]-benzoxazin-6-carbonsäureethylester;

3L-9,10-Difluor-3-phenyl-7-oxo-7H-pyrido-[1,2,3-de]-[1,4]-benzoxazin-6-carbonsäure;

3D-9,10-Difluor-3-phenyl-7-oxo-7H-pyrido-[1,2,3-de]-[1,4]-benzoxazin-6-carbonsäure;

3L-9-Fluor-10(4methyl-1-piperazinyl)-3-phenyl-7-oxo-7H-pyrido-[1,2,3-de][1,4]-benzoxazin-6-carbonsäure

und

3D-9-Fluor-10(4-methyl-1-piperazinyl)-3-phenyl-7-oxo-7H-pyrido-[1,2,3-de][1,4]-benzoxazin-6-carbonsäure.

5. Enantiomerenreine 1,8-verbrückte 4-Chinolon-3-carbonsäuren und Derivaten der Formel (I)

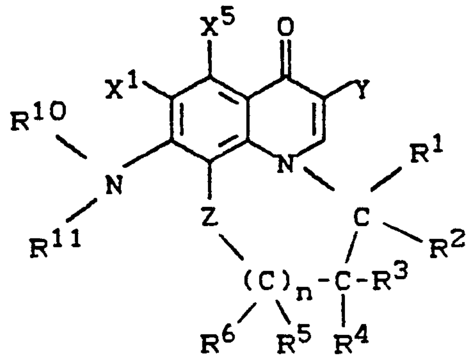

(I)

in der

Y eine Carboxylgruppe, eine Nitrilgruppe, eine Estergruppe $-COOR^7$ oder eine Säureamidgruppe $-CONR^8R^9$ darstellt, wobei $R^7$ für $C_1$-$C_4$-Alkyl steht, $R^8$ und $R^9$ für Wasserstoff oder $C_1$-$C_4$-Alkyl stehen, $R^9$ außerdem gegebenenfalls substituiertes Phenyl sein kann,

$X^1$ für Wasserstoff, Nitro, Alkyl mit 1 bis 3 Kohlenstoffatomen, Halogen, bevorzugt Fluor steht,

$X^5$ Wasserstoff, Halogen oder Methyl sein kann,

$R^{10}$ und $R^{11}$ gemeinsam mit dem Stickstoffatom, an das sie gebunden sind, einen 5- oder 6-gliedrigen heterocyclischen Ring bilden, der als Ringglied zusätzlich die Atome oder Gruppen -O-, -S-, -SO-, $-SO_2-$,

oder -CO-N-$R^{12}$ enthalten kann und der gegebenenfalls an den Kohlenstoffatomen ein- bis dreifach durch $C_1$-$C_4$-Alkyl, gegebenenfalls durch Chlor, Fluor, Brom, Methyl, Phenyl, Hydroxy, Methoxy, Benzyloxy, Nitro oder Piperidino ein- bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und Ethylaminomethyl substituiert sein kann, wobei

$R^{12}$ für Wasserstoff, eine verzweigte oder unverzweigte Alkyl-, Alkenyl- oder Alkinylgruppe mit 1 bis 6 Kohlenstoffatomen, die gegebenenfalls durch ein oder zwei Hydroxy-, Alkoxy-, Alkylamino- oder Dialkylaminogruppen mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, eine gegebenenfalls in Phenylrest substituierte Phenylalkylgruppe mit bis zu 4 Kohlenstoffatomen im aliphatischen Teil,

einen gegebenenfalls durch Hydroxy, Methoxy, Chlor oder Fluor ein- oder zweifach substituierten Phenacylrest, einen Oxoalkylrest mit bis zu 6 Kohlenstoffatomen steht, ferner einen Rest $COR^{13}$ oder $SO_2R^{14}$ bedeutet, wobei

$R^{13}$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Alkoxy mit 1 bis 4 Kohlenstoffatomen, Amino, Alkylamino oder Dialkylamino mit 1 bis 5 Kohlenstoffatomen im Alkylteil und

$R^{14}$ geradkettiges oder verzweigtes Alkyl mit 1 bis 3 Kohlenstoffatomen darstellt, und

Z für Sauerstoff, einen Aminrest $NR^{15}$ steht, wobei $R^{15}$ Wasserstoff, einen gegebenenfalls durch Halogen, Trifluormethyl, Nitro, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen, einen gegebenenfalls durch Halogen, eine Nitrogruppe, oder Alkylgruppe mit 1-3 Kohlenstoffatomen, eine Alkoxy- bzw. Alkylmercaptogruppe mit jeweils 1-3 Kohlenstoffatomen substituierten Phenylrest bedeutet, sowie ferner einen Acylrest $R^{16}$-CO-bzw. $R^{17}SO_2$- darstellt, wobei $R^{16}$ bzw. $R^{17}$ Alkylreste mit 1-6 Kohlenstoffatomen oder gegebenenfalls substituierte Phenylreste darstellen, sowie ein

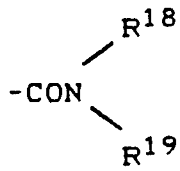 oder 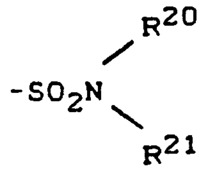

-Rest sein kann, wobei die Reste $R^{18}$ bis $R^{21}$ Wasserstoff, Alkyl mit 1-6 Kohlenstoffatomen oder einen gegebenenfalls substituierten Phenylrest darstellen

und

$R^1$, $R^2$, $R^3$, $R^4$, $R^5$ und $R^6$ für Wasserstoff, eine gegebenenfalls ein-oder mehrfach durch Halogen, insbesondere Chlor oder Fluor, ferner Nitro, Cyano, Hydroxy, Alkoxy, bzw. Alkylmercapto mit 1-3 Kohlenstoffatomen im Alkoholteil substituierte Alkylgruppe mit 1-6 Kohlenstoffatomen, einen gegebenenfalls durch Halogen, Nitro, Alkyl bzw. Alkoxy bzw. Alkylmercapto mit jeweils bis zu 3 Kohlenstoffatomen, Hydroxy, Aryloxy, Arylthio, Cyano oder einen Esterrest mit 1-3 Kohlenstoffatomen im Alkoholteil substituierten Phenylrest, Naphthylrest oder heterocyclischen Rest wie z.B. Thiophen-, Furan-, Pyrrol-, Thiazol-, Pyridin- oder Pyrimidin-Rest stehen mit der Maßgabe, daß $R^1$ und $R^2$ und/oder $R^3$ und $R^4$ und/oder $R^5$ und $R^6$ verschieden sind, sowie $R^2$ mit $R^3$ und/oder $R^4$ mit $R^5$ jeweils mit dem Kohlenstoffatomen, an das sie gebunden sind, einen 3-7 gliedrigen Ring bilden, der gegebenenfalls durch gegebenenfalls substituierte Alkylreste mit 1-3 Kohlenstoffatomen oder gegebenenfalls substituierte Arylreste substituiert ist und

n = 0 oder 1 bedeutet,

und deren pharmazeutisch verwendbare Hydrate, Alkali-, Erdalkali-, Silber- und Guanidiniumsalze, sowie ihre Ester zur Anwendung in einem Verfahren zur therapeutischen Behandlung des menschlischen oder tierischen Körpers.

6. Verfahren zur Herstellung von enantiomerenreinen 1,8-verbrückten 4-Chinolon-3-carbonsäuren und Derivaten der Formel (I)

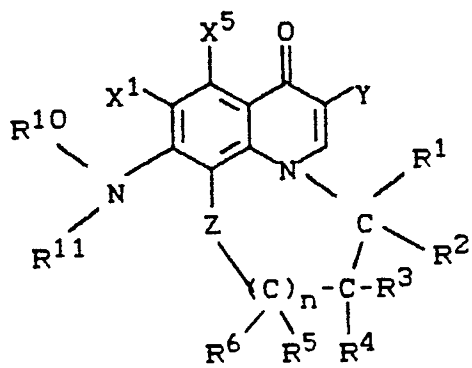

(I)

in der

Y eine Carboxylgruppe, eine Nitrilgruppe, eine Estergruppe $-COOR^7$ oder eine Säureamidgruppe $-CONR^8R^9$ darstellt, wobei $R^7$ für $C_1$-$C_4$-Alkyl steht, $R^8$ und $R^9$ für Wasserstoff oder $C_1$-$C_4$-Alkyl stehen, $R^9$ außerdem gegebenenfalls substituiertes Phenyl sein kann,

$X^1$ für Wasserstoff, Nitro, Alkyl mit 1 bis 3 Kohlenstoffatomen, Halogen, bevorzugt Fluor steht,

$X^5$ Wasserstoff, Halogen oder Methyl sein kann,

$R^{10}$ und $R^{11}$ gemeinsam mit dem Stickstoffatom, an das sie gebunden sind, einen 5- oder 6-gliedrigen heterocyclischen Ring bilden, der als Ringglied zusätzlich

die Atome oder Gruppen -O-, -S-, -SO-, $-SO_2-$,

$>N-R^{12}$

oder $-CO-\overset{|}{N}-R^{12}$ enthalten kann und der gegebenenfalls an den Kohlenstoffatomen ein- bis dreifach durch $C_1$-$C_4$-Alkyl, gegebenenfalls durch Chlor, Fluor, Brom, Methyl, Phenyl, Hydroxy, Methoxy, Benzyloxy, Nitro oder Piperidino ein- bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und Ethylaminomethyl substituiert sein kann, wobei

$R^{12}$ für Wasserstoff, eine verzweigte oder unverzweigte Alkyl-, Alkenyl- oder Alkinylgruppe mit 1 bis 6 Kohlenstoffatomen, die gegebenenfalls durch ein oder zwei Hydroxy-, Alkoxy-, Alkylamino- oder Dialkylaminogruppen mit 1 bis 3 Kohlenstoffatomen für einen Alkylrest, die

Cyangruppe, die Alkoxycarbonylgruppe mit 1 bis 4 Kohlenstoffatomen im Alkoholteil substituiert sein kann, eine gegebenenfalls in Phenylrest substituierte Phenylalkylgruppe mit bis zu 4 Kohlenstoffatomen im aliphatischen Teil,

einen gegebenenfalls durch Hydroxy, Methoxy, Chlor oder Fluor ein- oder zweifach substituierten Phenacrylrest, einen Oxoalkylrest mit bis zu 6 Kohlenstoffatomen steht, ferner einen Rest $COR^{13}$ oder $SO_2R^{14}$ bedeutet, wobei

$R^{13}$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Alkoxy mit 1 bis 4 Kohlenstoffatomen, Amino, Alkylamino oder Dialkylamino mit 1 bis 5 Kohlenstoffatomen im Alkylteil und

$R^{14}$ geradkettiges oder verzweigtes Alkyl mit 1 bis 3 Kohlenstoffatomen darstellt, und

Z für Sauerstoff, einen Aminrest $NR^{15}$ steht, wobei $R^{15}$ Wasserstoff, einen gegebenenfalls durch Halogen, Trifluormethyl, Nitro, Cyano, Hydroxy, Alkoxy bzw. Alkylmercapto mit 1-3 Kohlenstoffatomen, Aryloxy, Arylthio oder Esterrest mit 1-3

Kohlenstoffatomen im Alkoholteil substituierten Alkylrest mit 1-6 Kohlenstoffatomen, einen gegebenenfalls durch Halogen, eine Nitrogruppe, oder Alkylgruppe mit 1-3 Kohlenstoffatomen, eine Alkoxy- bzw. Alkylmercaptogruppe mit jeweils 1-3 Kohlenstoffatomen substituierten Phenylrest bedeutet, sowie ferner einen Acylrest $R^{16}$-CO-bzw. $R^{17}SO_2$- darstellt, wobei $R^{16}$ bzw. $R^{17}$ Alkylreste mit 1-6 Kohlenstoffatomen oder gegebenenfalls substituierte Phenylreste darstellen, sowie ein

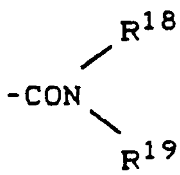

oder

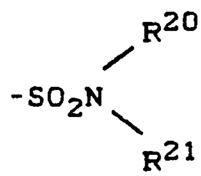

-Rest sein kann, wobei die Reste $R^{18}$ bis $R^{21}$ Wasserstoff, Alkyl mit 1-6 Kohlenstoffatomen oder einen gegebenenfalls substituierten Phenylrest darstellen

und

$R^1$, $R^2$, $R^3$, $R^4$, $R^5$ und $R^6$ für Wasserstoff, eine gegebenenfalls ein-oder mehrfach durch Halogen, insbesondere Chlor oder Fluor, ferner Nitro, Cyano, Hydroxy, Alkoxy, bzw. Alkylmercapto mit 1-3 Kohlenstoffatomen im Alkoholteil substituierte Alkylgruppe mit 1-6 Kohlenstoffatomen, einen gegebenenfalls durch Halogen, Nitro, Alkyl bzw. Alkoxy bzw. Alkylmercapto mit jeweils bis zu 3 Kohlenstoffatomen, Hydroxy, Aryloxy, Arylthio,

Cyano oder einen Esterrest mit 1-3 Kohlenstoffatomen im Alkoholteil substituierten Phenylrest, Naphthylrest oder heterocyclischen Rest wie z.B. Thiophen-, Furan-, Pyrrol-, Thiazol-, Pyridin- oder Pyrimidin-Rest stehen mit der Maßgabe, daß $R^1$ und $R^2$ und/oder $R^3$ und $R^4$ und/oder $R^5$ und $R^6$ verschieden sind, sowie $R^2$ mit $R^3$ und/oder $R^4$ mit $R^5$ jeweils mit dem Kohlenstoffatomen, an das sie gebunden sind, einen 3-7 gliedrigen Ring bilden, der gegebenenfalls durch gegebenenfalls substituierte Alkylreste mit 1-3 Kohlenstoffatomen oder gegebenenfalls substituierte Arylreste substituiert ist und

n = 0 oder 1 bedeutet,

und deren pharmazeutisch verwendbaren Hydraten, Alkali-, Erdalkali-, Silber- und Guanidiniumsalzen, sowie ihren Estern,

dadurch gekennzeichnet, daß man Chinoloncarbonsäurederivate der Formel (II)

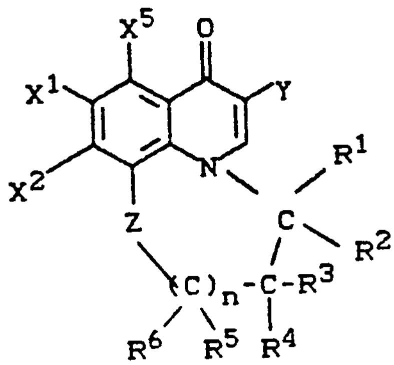

(II)

in der die Reste $X^1$, $X^5$, $R^1$-$R^6$, Z, Y sowie n die oben angegebene Bedeutung haben, sowie $X^2$ für Chlor und Fluor steht,

mit Aminen der Formel (III)

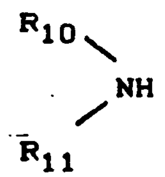

(III)

in welcher

$R_{10}$ und $R_{11}$ die oben angegebene Bedeutung haben,

gegebenenfalls in Gegenwart von Säurebindern umsetzt.

7. Verfahren zur Herstellung von enantiomerenreinen 1,8-verbrückten 4-Chinolon-3-carbonsäuren und Derivaten der Formel (I) gemäß Anspruch 6, dadurch gekennzeichnet, daß man eine 10-(1-Piperazinyl)-verbindung (bei n=O) bzw. 11-(1-Piperazinyl)verbindung (bei n=1) der Formel (IV)

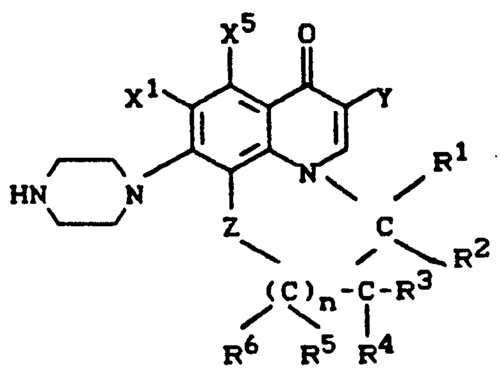

(IV)

in welcher

$X^1$, $X^5$, $R^1$-$R^6$, Z und Y sowie n die oben angegebene Bedeutung haben und

der Piperazinylrest an den Kohlenstoffatomen 1-3 fach durch $C_1$-$C_4$-Alkyl, 2-Thienyl oder gegebenenfalls substituiertes Cyclohexyl oder Phenyl substituiert sein kann mit Verbindungen der Formel (V)

$$R^{12}X \qquad (V)$$

in welcher

$R^{12}$ die oben angegebene Bedeutung hat, jedoch nicht Wasserstoff sein kann, und

X Fluor, Chlor, Brom, Iod, Hydroxy, Acyloxy, Ethoxy, Phenoxy, 4-Nitrophenoxy bedeutet,

gegebenenfalls in Gegenwart von Säurebindern umsetzt.

8. Verfahren zur Herstellung von enantiomerenreinen 1,8-verbrückten 4-Chinolon-4-carbonsäuren und Derivaten der Formel (I) gemäß Anspruch 6, dadurch gekennzeichnet, daß man 10-(1-Piperazinyl)chinoloncarbonsäurederivate (n=0) bzw. 11-(1-Piperazinyl)chinoloncarbonsäurederivate (n=1) der Formel (IV) nach Anspruch 7, in welcher der Piperazinylrest an den Kohlenstoffatomen 1-3fach durch $C_1$-$C_4$-Alkyl, 2-Thienyl oder gegebenenfalls substituiertes Cyclohexyl oder Phenyl substituiert sein kann, mit Micheal-Acceptoren oder Formel (VI),

$$B\text{-}CH{=}CH_2 \qquad (VI)$$

in der

B für CN, CO-$R^{22}$ oder COOR$^{23}$ steht, wobei $R^{22}$ für Methyl oder Ethyl und $R^{23}$ für Methyl, Ethyl, n- oder i-Propyl steht, umsetzt.

9. Arzneimittel, enthaltend enantiomerenreine 1,8-verbrückte 4-Chinolon-3-carbonsäuren und Derivaten der Formel (I)

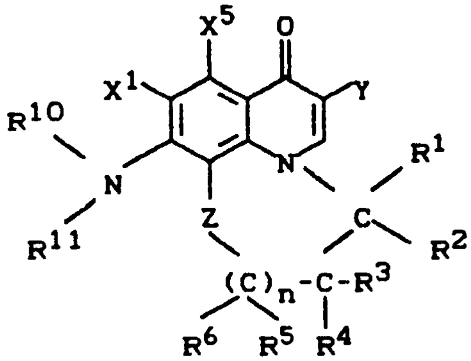

(I)

in der

Y eine Carboxylgruppe, eine Nitrilgruppe, eine Estergruppe -COOR$^7$ oder eine Säureamidgruppe -CONR$^8$R$^9$ darstellt, wobei $R^7$ für $C_1$-$C_4$-Alkyl steht, $R^8$ und $R^9$ für Wasserstoff oder $C_1$-$C_4$-Alkyl stehen, $R^9$ außerdem gegebenenfalls substituiertes Phenyl sein kann,

$X^1$ für Wasserstoff, Nitro, Alkyl mit 1 bis 3 Kohlenstoffatomen, Halogen, bevorzugt Fluor steht,

$X^5$ Wasserstoff, Halogen oder Methyl sein kann,

$R^{10}$ und $R^{11}$ gemeinsam mit dem Stickstoffatom, an das sie gebunden sind, einen 5- oder 6-gliedrigen heterocyclischen Ring bilden, der als Ringglied zusätzlich

die Atome oder Gruppen -O-, -S-, -SO-, $-SO_2-$,

$>N-R^{12}$

|

oder $-CO-N-R^{12}$ enthalten kann und der gegebenenfalls an den Kohlenstoffatomen ein- bis dreifach durch $C_1$-$C_4$-Alkyl, gegebenenfalls durch Chlor, Fluor, Brom, Methyl, Phenyl, Hydroxy, Methoxy, Benzyloxy, Nitro oder Piperidino ein- bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und Ethylaminomethyl substituiert sein kann, wobei

$R^{12}$ für Wasserstoff, eine verzweigte oder unverzweigte Alkyl-, Alkenyl- oder Alkinylgruppe mit 1 bis 6 Kohlenstoffatomen, die gegebenenfalls durch ein oder zwei Hydroxy-, Alkoxy-, Alkylamino- oder Dialkylaminogruppen mit 1 bis 3 Kohlenstoffatomen für einen Alkylrest, die

Cyangruppe, die Alkoxycarbonylgruppe mit 1 bis 4 Kohlenstoffatomen im Alkoholteil substituiert sein kann, eine gegebenenfalls in Phenylrest substituierte Phenylalkylgruppe mit bis zu 4 Kohlenstoffatomen im aliphatischen Teil,

einen gegebenenfalls durch Hydroxy, Methoxy, Chlor oder Fluor ein- oder zweifach substituierten Phenacylrest, einen Oxoalkylrest mit bis zu 6 Kohlenstoffatomen steht, ferner einen Rest $COR^{13}$ oder $SO_2R^{14}$ bedeutet, wobei

$R^{13}$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Alkoxy mit 1 bis 4 Kohlenstoffatomen, Amino, Alkylamino oder Dialkylamino mit 1 bis 5 Kohlenstoffatomen im Alkylteil und

$R^{14}$ geradkettiges oder verzweigtes Alkyl mit 1 bis 3 Kohlenstoffatomen darstellt, und

Z für Sauerstoff, einen Aminrest $NR^{15}$ steht, wobei $R^{15}$ Wasserstoff, einen gegebenenfalls durch Halogen, Trifluormethyl, Nitro, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen, einen gegebenenfalls durch Halogen, eine Nitrogruppe, oder Alkylgruppe mit 1-3 Kohlenstoffatomen, eine Alkoxy- bzw. Alkylmercaptogruppe mit jeweils 1-3 Kohlenstoffatomen substituierten Phenylrest bedeutet, sowie ferner einen Acylrest $R^{16}$-CO-bzw. $R^{17}SO_2$- darstellt, wobei $R^{16}$ bzw. $R^{17}$ Alkylreste mit 1-6 Kohlenstoffatomen oder gegebenenfalls substituierte Phenylreste darstellen, sowie ein

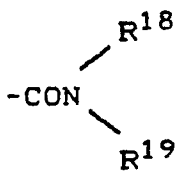

oder

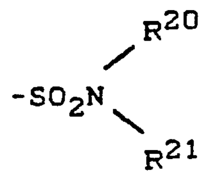

-Rest sein kann, wobei die Reste $R^{18}$ bis $R^{21}$ Wasserstoff, Alkyl mit 1-6 Kohlenstoffatomen oder einen gegebenenfalls substituierten Phenylrest darstellen

und

$R^1$, $R^2$, $R^3$, $R^4$, $R^5$ und $R^6$ für Wasserstoff, eine gegebenenfalls ein-oder mehrfach durch Halogen, insbesondere Chlor oder Fluor, ferner Nitro, Cyano, Hydroxy, Alkoxy, bzw. Alkylmercapto mit 1-3 Kohlenstoffatomen im Alkoholteil substituierte Alkylgruppe mit 1-6 Kohlenstoffatomen, einen gegebenenfalls durch Halogen, Nitro, Alkyl bzw. Alkoxy bzw. Alkylmercapto mit jeweils bis zu 3 Kohlenstoffatomen, Hydroxy, Aryloxy, Arylthio, Cyano oder einen Esterrest mit 1-3 Kohlenstoffatomen im Alkoholteil substituierten Phenylrest, Naphthylrest oder heterocyclischen Rest wie z.B. Thiophen-, Furan-, Pyrrol-, Thiazol-, Pyridin- oder Pyrimidin-Rest stehen mit der Maßgabe, daß $R^1$ und $R^2$ und/oder $R^3$ und $R^4$ und/oder $R^5$ und $R^6$ verschieden sind, sowie $R^2$ mit $R^3$ und/oder $R^4$ mit $R^5$ jeweils mit dem Kohlenstoffatomen, an das sie gebunden sind, einen 3-7 gliedrigen Ring bilden, der gegebenenfalls durch gegebenenfalls substituierte Alkylreste mit 1-3 Kohlenstoffatomen oder gegebenenfalls substituierte Arylreste substituiert ist und

n = 0 oder 1 bedeutet,

und deren pharmazeutisch verwendbare Hydrate, Alkali-, Erdalkali-, Silber- und Guanidiniumsalze, sowie ihre Ester.

10. Verwendung von enantiomerenreinen 1,8-verbrückten 4-Chinolon-3-carbonsäuren und Derivaten der Formel (I)

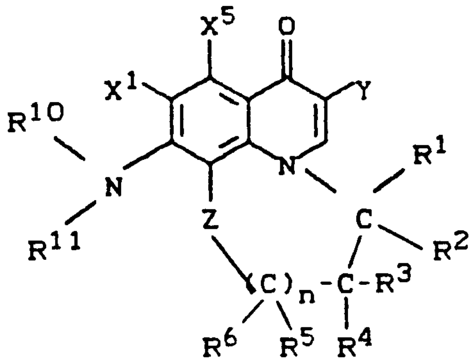

(I)

in der

Y eine Carboxylgruppe, eine Nitrilgruppe, eine Estergruppe $-COOR^7$ oder eine Säureamidgruppe $-CONR^8R^9$ darstellt, wobei $R^7$ für $C_1$-$C_4$-Alkyl steht, $R^8$ und $R^9$ für Wasserstoff oder $C_1$-$C_4$-Alkyl stehen, $R^9$ außerdem gegebenenfalls substituiertes Phenyl sein kann,

$X^1$ für Wasserstoff, Nitro, Alkyl mit 1 bis 3 Kohlenstoffatomen, Halogen, bevorzugt Fluor steht,

$X^5$ Wasserstoff, Halogen oder Methyl sein kann,

$R^{10}$ und $R^{11}$ gemeinsam mit dem Stickstoffatom, an das sie gebunden sind, einen 5- oder 6-gliedrigen heterocyclischen Ring bilden, der als Ringglied zusätzlich die Atome oder Gruppen -O-, -S-, -SO-, $-SO_2-$,

$>N-R^{12}$

|

oder $-CO-N-R^{12}$ enthalten kann und der gegebenenfalls an den Kohlenstoffatomen ein- bis dreifach durch $C_1$-$C_4$-Alkyl, gegebenenfalls durch Chlor, Fluor, Brom, Methyl, Phenyl, Hydroxy, Methoxy, Benzyloxy, Nitro oder Piperidino ein- bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und

Ethylaminomethyl substituiert sein kann, wobei

$R^{12}$ für Wasserstoff, eine verzweigte oder unverzweigte Alkyl-, Alkenyl- oder Alkinylgruppe mit 1 bis 6 Kohlenstoffatomen, die gegebenenfalls durch ein oder zwei Hydroxy-, Alkoxy-, Alkylamino- oder Dialkylaminogruppen mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, eine gegebenenfalls in Phenylrest substituierte Phenylalkylgruppe mit bis zu 4 Kohlenstoffatomen im aliphatischen Teil,

einen gegebenenfalls durch Hydroxy, Methoxy, Chlor oder Fluor ein- oder zweifach substituierten Phenacrylrest, einen Oxoalkylrest mit bis zu 6 Kohlenstoffatomen steht, ferner einen Rest $COR^{13}$ oder $SO_2R^{14}$ bedeutet, wobei

$R^{13}$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Alkoxy mit 1 bis 4 Kohlenstoffatomen, Amino, Alkylamino oder Dialkylamino mit 1 bis 5 Kohlenstoffatomen im Alkylteil und

$R^{14}$ geradkettiges oder verzweigtes Alkyl mit 1 bis 3 Kohlenstoffatomen darstellt, und

Z für Sauerstoff, einen Aminrest $NR^{15}$ steht, wobei $R^{15}$ Wasserstoff, einen gegebenenfalls durch Halogen, Trifluormethyl, Nitro, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen, einen gegebenenfalls durch Halogen, eine Nitrogruppe, oder Alkylgruppe mit 1-3 Kohlenstoffatomen, eine Alkoxy- bzw. Alkylmercaptogruppe mit jeweils 1-3 Kohlenstoffatomen substituierten Phenylrest bedeutet, sowie ferner einen Acylrest $R^{16}$-CO-bzw. $R^{17}SO_2$- darstellt, wobei $R^{16}$ bzw. $R^{17}$ Alkylreste mit 1-6 Kohlenstoffatomen oder gegebenenfalls substituierte Phenylreste darstellen, sowie ein

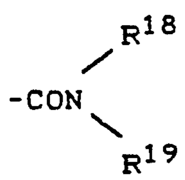 oder 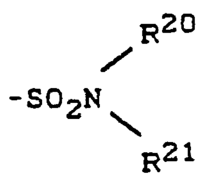

-Rest sein kann, wobei die Reste $R^{18}$ bis $R^{21}$ Wasserstoff, Alkyl mit 1-6 Kohlenstoffatomen oder einen gegebenenfalls substituierten Phenylrest darstellen

und

$R^1$, $R^2$, $R^3$, $R^4$, $R^5$ und $R^6$ für Wasserstoff, eine gegebenenfalls ein-oder mehrfach durch Halogen,

insbesondere Chlor oder Fluor, ferner Nitro, Cyano, Hydroxy, Alkoxy, bzw. Alkylmercapto mit 1-3 Kohlenstoffatomen im Alkoholteil substituierte Alkylgruppe mit 1-6 Kohlenstoffatomen, einen gegebenenfalls durch Halogen, Nitro, Alkyl bzw. Alkoxy bzw. Alkylmercapto mit jeweils bis zu 3 Kohlenstoffatomen, Hydroxy, Aryloxy, Arylthio, Cyano oder einen Esterrest mit 1-3 Kohlenstoffatomen im Alkoholteil substituierten Phenylrest, Naphthylrest oder heterocyclischen Rest wie z.B. Thiophen-, Furan-, Pyrrol-, Thiazol-, Pyridin- oder Pyrimidin-Rest stehen mit der Maßgabe, daß $R^1$ und $R^2$ und/oder $R^3$ und $R^4$ und/oder $R^5$ und $R^6$ verschieden sind, sowie $R^2$ mit $R^3$ und/oder $R^4$ mit $R^5$ jeweils mit dem Kohlenstoffatomen, an das sie gebunden sind, einen 3-7 gliedrigen Ring bilden, der gegebenenfalls durch gegebenenfalls substituierte Alkylreste mit 1-3 Kohlenstoffatomen oder gegebenenfalls substituierte Arylreste substituiert ist und

n = 0 oder 1 bedeutet,

und deren pharmazeutisch verwendbare Hydraten, Alkali-, Erdalkali-, Silber- und Guanidiniumsalzen, sowie ihren Estern zur Herstellung von Arzneimitteln.